# EUROPEAN PATENT APPLICATION

(11) **EP 4 559 466 A1**
(43) Date of publication of application: **28.05.2025**
(21) Application number: 23383203.9
(22) Date of filing: 23.11.2023
(51) Int. Cl.: A61K 31/7004, A61K 31/7016, A61P 25/30, G01N 33/50

(54) **MICROBIOTA SIGNATURE TO DETECT FOOD ADDICTION AND TREATMENT THEREOF**

(71) Applicant: Universitat Pompeu Fabra (UPF), 08002 Barcelona (ES); Fundació Institut d'Investigació Biomèdica de Girona Dr. Josep Trueta (IDIBGI), 17190 Salt (Girona) (ES); Vilnius University, 10257 Vilnius (LT)
(72) Inventor: MALDONADO LÓPEZ, Rafael, Barcelona (ES); MARTÍN GARCÍA, Elena, Barcelona (ES); SAMULENAITE, Soveiga, Barcelona (ES); BUROKAS, Aurelijus, Vilnius (LT); FERNÁNDEZ REAL, José Manuel, Salt (ES); MAYNERIS, Jordi, Salt (ES)
(74) Representative: Cueto, Sénida

(57) **Abstract**

The present invention relates to a combined preparation of rhamnose and lactulose for use in the treatment and/or prevention of food addiction in a subject and a microbiota signature to detect food addiction the comprises detecting the levels of bacteria from *Enterorhabdus, Lachnospiracceae, Allobaculum, Blautia, Anaeroplasma* and *Gastranaerophilales* from a biological sample

## Description

### TECHNICAL FIELD

The present invention belongs to the medical field of food addiction, and relates to a microbiota signature to detect a subject suffering of food addiction and a combined preparation comprising lactulose and rhamnose for use in the treatment thereof.

### BACKGROUND

The gut microbiota is integrated by a large variety of microbes, including bacteria, fungi, archaea, and viruses that colonize the digestive tract. These microorganisms offer numerous benefits by interacting with the host and establishing a symbiotic relationship. This symbiosis is not only essential for peripheral physiological functions, but also affects neurobiological processes. Interestingly, the relationships between addiction and dysbiosis in gut microbiota are gaining high relevance, mainly in alcohol abuse.

Food addiction is a complex multifactorial behavioral disorder characterized by a loss of control over food intake that has increased its prevalence in recent years. It is characterized by the compulsive intake of palatable foods, which can produce adaptive changes in the reward brain network. This behavioral alteration is related to obesity and other eating disorders, and lacks effective treatment, leading to high socio-economical costs worldwide. A widely accepted instrument currently used in the clinic to evaluate food addiction is the Yale Food Addiction Scale (YFAS), updated in 2016 to YFAS 2.0 to apply the 5th edition of the Diagnostic and Statistical Manual of Mental Disorders (DSM-5) criteria for substance use disorder to food addiction (Gearhardt AN, Corbin WR, Brownell KD. Development of the Yale Food Addiction Scale Version 2.0. Psychol Addict Behav 2016;30:113-21. doi:10.1037/adb0000136). The YFAS 2.0 food addiction criteria can be summarized in three hallmarks also used in rodent models to mimic this disorder: persistent food-seeking, high motivation to obtain food, and compulsivity-like behavior [Maldonado R, Calvé P, Garcia-Blanco A, et al. Vulnerability to addiction. Neuropharmacology 2021;186:108466. doi:10.1016/j.neuropharm.2021.108466].

The study of the gut microbiota signatures for vulnerability to food addiction has gained attention in recent years, although no direct relationships have been yet established. Indeed, environmental factors and dietary patterns have a major influence on the gut microbiota composition, and the overconsumption of highly palatable food may promote a gut microbiota dysbiosis that has been recently proposed to participate in the loss of eating control. In agreement, individuals with obesity, which may be promoted by food addiction, showed altered gut microbiota with a reduced variety that facilitates energy absorption capacity and may affect host brain function. However, the involvement of gut microbiota in the loss of eating control and food addiction has not yet been demonstrated.

The patent document WO2021030091A1 discloses a microbiota signature to differentiate between obese and non-obese patients. However, this signature differs from the present invention as it does not disclose the same microorganisms and has a different etiology. Furthermore, this document does not disclose the combined preparation of the present invention, nor its mechanism of action.

The inventors of the present specification have obtained extreme subpopulations of food-addicted and non-addicted mice to identify the differential gut microbiota signatures associated with vulnerability to addiction. Using parallel food addiction-like criteria, the YFAS 2.0 score was applied to classify a cohort of patients to assess the gut microbiota signatures of this behavioral disorder as biomarkers. Next, the role of one of the microorganisms identified: *Blautia,* was functionally validated by administering the combined preparation of the invention: the prebiotics lactulose and rhamnose. Both prebiotics synergistically increased *Blautia* abundance and surprisingly prevented food addiction development in mice.

The scientific article Reichardt N, Vollmer M, Holtrop G, et al. Specific substrate-driven changes in human faecal microbiota composition contrast with functional redundancy in short-chain fatty acid production. ISME J. 2018;12(2):610-622. doi:10.1038/ismej.2017.196 discloses that the prebiotic rhamnose increases the amount of *Blautia* in mice feaces

The scientific article Cui S, Gu J, Liu X, et al. Lactulose significantly increased the relative abundance of Bifidobacterium and Blautia in mice feces, as revealed by 16S rRNA amplicon sequencing. J Sci Food Agric. 2021;101(13):5721-5729. doi:10.1002/jsfa.11181 discloses that the prebiotic lactulose increases the amount of *Blautia* in mice feaces.

However, none of the documents mention the other prebiotic, neither the synergistic effect when combined or the prevention of food addiction development. The inventors of the present specification have identified a surprising synergy when both prebiotics were administered as part of a combined preparation.

### DESCRIPTION

A first object of the invention relates to a combined preparation of rhamnose and lactulose for use in the treatment and /or prevention of food addiction in a subject.

The administration to the subject can be simultaneous, sequential or separate, preferably simultaneous.

Lactulose is a sugar of chemical 4-O-β-D-galactopyranosyl-D-fructofuranose. The molecular formula is C12H22O11.

Rhamnose is a sugar with a chemical name (3R,4R,5R,6S)-6-methyloxane-2,3,4,5-tetrol. The molecular formula is C6H12O5.

As used herein, the term "a combination of," or "a combined preparation" when referring to two or more compounds, agents, or additional active pharmaceutical ingredients, means the administration of two or more compounds, agents, or active pharmaceutical ingredients to the subject prior to, concurrent with, or subsequent to each other.

The combined preparation of the invention shows synergy in the treatment and/or prevention of food addiction. This is, there is an increased therapeutic effect when lactulose and rhamnose are administered as a composition.

Food addiction may lead to obesity, diabetes and/or metabolic syndrome. Therefore, in a particular embodiment of the invention, food addiction comprises obesity.

In another particular embodiment, the treatment and/or prevention of food addiction comprises alleviation of one or more of the following symptoms: persistent food-seeking, high motivation to obtain food, and compulsivity-like behavior. As will be shown in the examples of the invention, the combined preparation of the invention is quite effective in the treatment of those symptoms.

The term "preventing," as used herein, refers to administering a combined preparation prior to the onset of clinical symptoms of a disease or conditions so as to prevent a physical manifestation of aberrations associated with the disease or condition. In the context of food addiction, the term "preventing" refers to administering a combined preparation prior to the onset of clinical symptoms of food addiction so as to prevent a physical manifestation of aberrations associated with food addiction, such as the addictive-like behavior, for example: persistent food-seeking, high motivation to obtain food, and compulsivity-like behavior.

The terms "treatment" and "treating" as used herein refer to the medical management of a subject with the intent to cure, ameliorate, stabilize, or prevent a disease, pathological condition, or disorder. This term includes active treatment, that is, treatment directed specifically toward the improvement of a disease, pathological condition, or disorder, and also includes causal treatment, that is, treatment directed toward removal of the cause of the associated disease, pathological condition, or disorder. In addition, this term includes palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder. It is understood that treatment, while intended to cure, ameliorate, stabilize, or prevent a disease, pathological condition, or disorder, need not actually result in the cure, amelioration, stabilization or prevention. The effects of treatment can be measured or assessed as described herein and as known in the art as is suitable for the disease, pathological condition, or disorder involved. Such measurements and assessments can be made in qualitative and/or quantitative terms. Thus, for example, characteristics or features of a disease, pathological condition, or disorder and/or symptoms of a disease, pathological condition, or disorder can be reduced to any effect or to any amount. In the context of a subject suffering from food addiction, the terms "treatment" and "treating" refer to the medical management of a subject with the intent to cure, ameliorate, or stabilize food addiction. In the context of a subject at risk of developing food addiction, the terms "treatment" and "treating" refer to the medical management of a subject with the intent to prevent food addiction.

As used herein, "subject" includes, but is not limited to, animals. The subject can be a vertebrate, more specifically a mammal (e.g., a human, horse, pig, rabbit, dog, sheep, goat, non-human primate, cow, cat, guinea pig or rodent). The term does not denote a particular age or sex. Thus, adult and newborn subjects, as well as fetuses, whether male or female, are intended to be covered. A patient refers to a subject afflicted with a disease or disorder. The term "patient" includes human and veterinary subjects. In one aspect, the compounds described herein can be administered to a subject comprising a human or an animal including, but not limited to, a mouse, dog, cat, horse, bovine or ovine and the like, that is in need of prevention, alleviation or amelioration from food addiction.

In a particular embodiment the subject is male.

The combined preparation can be administered to individuals aged from birth, 1 year or more, preferably 5 years or more, more preferably 10 years or more, or 20 years or more, or 30 years or more, or 40 years or more, or 50 years or more or 60 years or more, or 70 years or more or 80 years or more.

Regarding the dosage of the combined preparation of the invention, in a particular embodiment, the amount of lactulose and rhamnose is approximately the same.

In another particular embodiment the ratio of lactulose:rhamnose is from 100:1 to 2:1, or from 90:1 to 2:1, or from 80:1 to 2:1, or from 70:1 to 2:1, or from 60:1 to 2:1, or from 50:1 to 2:1, or from 40:1 to 2:1, or from 30:1 to 2:1, or from 20:1 to 2:1, or from 15:1 to 2:1, or from 14:1 to 2:1, or from 13:1 to 2:1, or from 12:1 to 2:1, or from 11:1 to 2:1, or from 10:1 to 2:1, or from 9:1 to 2:1, or from 8:1 to 2:1, or from 7:1 to 2:1, or from 6:1 to 2:1, or from 5:1 to 2:1, or from 4:1 to 2:1, or from 3:1 to 2:1, or from 2.8:1 to 1.1:1, or from 2.6:1 to 1.1:1, or from 2.4:1 to 1.1:1, or from 2.2:1 to 1.1:1, or from 2:1 to 1.1:1, or from 1.8:1 to 1.1:1, or from 1.6:1 to 1.1:1, or from 1.4:1 to 1.1:1, or from 1.3:1 to 1.1:1, or from 1.2:1 to 1.1:1

In another particular embodiment the ratio of rhamnose:lactulose is from 100:1 to 2:1, or from 90:1 to 2:1, or from 80:1 to 2:1, or from 70:1 to 2:1, or from 60:1 to 2:1, or from 50:1 to 2:1, or from 40:1 to 2:1, or from 30:1 to 2:1, or from 20:1 to 2:1, or from 15:1 to 2:1, or from 14:1 to 2:1, or from 13:1 to 2:1, or from 12:1 to 2:1, or from 11:1 to 2:1, or from 10:1 to 2:1, or from 9:1 to 2:1, or from 8:1 to 2:1, or from 7:1 to 2:1, or from 6:1 to 2:1, or from 5:1 to 2:1, or from 4:1 to 2:1, or from 3:1 to 2:1, or from 2.8:1 to 1.1:1, or from 2.6:1 to 1.1:1, or from 2.4:1 to 1.1:1, or from 2.2:1 to 1.1:1, or from 2:1 to 1.1:1, or from 1.8:1 to 1.1:1, or from 1.6:1 to 1.1:1, or from 1.4:1 to 1.1:1, or from 1.3:1 to 1.1:1, or from 1.2:1 to 1.1:1

For an appropriate administration of the combined preparation of the invention, it is necessary to dissolve it in water or any other liquid that does not affect the properties of the components of the composition prior to its intake by the subject. The combined composition of the invention can be dissolved in a liquid for an appropriate administration, the solvent being any drinkable liquid, preferably water.

In a particular embodiment, the amount of the prebiotic lactulose and rhamnose is substantially the same, for example, each of the prebiotics is 1% w/w of the total composition in a suitable drinkable liquid, such as water.

When the amount of both prebiotics is substantially the same, the concentration can be from 0.1 to 10 % w/w of the total composition in a suitable drinkable liquid, or from 0.2 to 9 % w/w, or from 0.3 to 8 % w/w, or from 0.4 to 7 % w/w, or from 0.5 to 6 % w/w, or from 0.6 to 5 % w/w, or from 0.7 to 4 % w/w, or from 0.8 to 2 % w/w in a suitable drinkable liquid such as water.

In another particular embodiment lactulose is from 0.1 to 10% w/w in water and rhamnose is from 0.1 to 10% w/w in water, or lactulose is from 0.2 to 7% w/w in water and rhamnose is from 0.2 to 7% w/w in water, or lactulose is from 0.3 to 5% w/w in water and rhamnose is from 0.3 to 5% w/w in water, or lactulose is from 0.4 to 6% w/w in water and rhamnose is from 0.5 to 6% w/w in water, or lactulose is from 0.5 to 5% w/w in water and rhamnose is from 0.5 to 5% w/w in water, or lactulose is from 0.6 to 4% w/w in water and rhamnose is from 0.6 to 4% w/w in water, or lactulose is from 0.7 to 3% w/w in water and rhamnose is from 0.7 to 3% w/w in water, or lactulose is from 0.8 to 2% w/w in water and rhamnose is from 0.8 to 2% w/w in water, lactulose is from 0.9 to 1.5% w/w in water and rhamnose is from 0.9 to 1.5% w/w in water, or lactulose is from 0.5 to 2% w/w in water and rhamnose is from 0.5 to 2% w/w in water. Preferably the amount of the prebiotic lactulose and rhamnose is substantially the same.

For example, an appropriate solvent may contain from about 0.001 to 500 mg/ml of lactulose and rhamnose, preferably, from 0.01 to 300 mg/ml, more preferably from 0.1 to 300 mg/ml, still more preferably from 0.2 to 200 mg/ml, still more preferably from 0.3 to 100 mg/ml, the solvent of lactulose and rhamnose being any drinkable liquid, preferably water.

The amount of the combined composition administered to a subject may be a dose of 1 mg/kg to 1000 mg/kg, preferably a dose of 10 mg/kg to 500 mg/kg, more preferably a dose of 50 mg/kg to 400 mg/kg, or even more preferably a dose of 100 mg/kg to 300 mg/kg.

Lactulose and/or rhamnose can be either extracted from non-digestible carbohydrate materials or synthetically produced.

The combined preparation can be administered with a variable administration interval, for example, from 1 to 7 times per week, preferably from 2 to 6 times per week, more preferably from 3 to 5 times per week.

In another particular embodiment, the combined preparation of the invention is administered orally, daily, for a period of time of between 1 day and 365 days, preferably between 1 day and 180 days, even more preferably between 1 day and 150 days, or between 1 day and 140 days, or between 1 day and 130 days, or between 1 day and 120 days, or between 1 day and 110 days, or between 1 day and 100 days, or between 1 day and 95 days, or between 1 day and 80 days, or between 1 day and 70 days, or between 1 day and 60 days, or between 1 day and 50 days, or between 1 day and 40 days, or between 1 day and 30 days, or between 1 day and 20 days,

Preferably the combined preparation of the invention is administered orally for 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, or 180 days

The combined preparation and pharmaceutical compositions described herein can be administered to the subject in a number of ways. Thus, for example, a combined preparation or pharmaceutical composition described herein can be administered to a subject vaginally, rectally, intranasally, orally, by inhalation, or parenterally, for example, by intradermal, subcutaneous, intramuscular, intraperitoneal, intrarectal, intraarterial, intralymphatic, intravenous, intrathecal and intratracheal routes. Parenteral administration, if used, is generally characterized by injection. Injectables can be prepared in conventional forms, either as liquid solutions or suspensions, solid forms suitable for solution or suspension in liquid prior to injection, or as emulsions. A more recently revised approach for parenteral administration involves the use of a slow-release or sustained-release system such that a constant dosage is maintained.

Preferably, the combined preparation and pharmaceutical compositions are administered orally and can include powders or granules, suspensions or solutions in water or non-aqueous media, capsules, sachets, or tablets. Thickeners, flavorings, diluents, emulsifiers, dispersing aids or binders can be desirable.

Another object of the invention relates to a method to detect food addiction in a subject comprising the detection of an amount of bacterial species selected from *Enterorhabdus, Lachnospiracceae, Allobaculum,* and *Blautia* from a first biological sample, wherein the amount of the bacterial species of *Enterorhabdus, Lachnospiracceae, Allobaculum,* and *Blautia* is reduced as compared to a reference value.

A skilled person would readily understand that *Enterorhabdus, Allobaculum,* and *Blautia* are genera of bacteria, while *Lachnospiracceae* is a family of bacteria. Thus, in the present specification, a microbiota signature means a bacteria signature.

As used herein, the term "reference value" refers to an individual with a body mass index (BMI) between 18.5 and 29.9 kg/m², preferably a BMI between 18.5 and 25 kg/m² Therefore, in the context of the present invention, the reference value means the amount of bacterial species selected from *Enterorhabdus, Lachnospiracceae, Allobaculum* and *Blautia* in a biological sample of a subject of a BMI between 18.5 and 29.9 kg/m²

In a particular embodiment, the species of the genera *Blautia* is one or more of the following species: *Blautia acetigignens, Blautia ammoniilytica, Blautia argi, Blautia brookingsii, Blautia caecimuris, Blautia celeris, Blautia coccoides, Blautia difficilis, Blautia faecicola, Blautia faecis, Blautia fusiformis, Blautia glucerasea, Blautia hansenii, Blautia hominis, Blautia hydrogenotrophica, Blautia intestinalis, Blautia lenta, Blautia liquoris, Blautia luti, Blautia marasmi, Blautia massiliensis (ex Durand et al. 2017), Blautia massiliensis (ex Liu et al. 2021), Blautia obeum, Blautia phocaeensis, Blautia product, Blautia provencensis, Blautia pseudococcoides, Blautia schinkii, Blautia segnis, Blautia stercoris, Blautia tarda, Blautia wexlerae, Candidatus Blautia avicola, Candidatus Blautia avistercoris, Candidatus Blautia excrementigallinarum, Candidatus Blautia excrementipullorum, Candidatus Blautia faecavium, Candidatus Blautia faecigallinarum, Candidatus Blautia faecipullorum, Candidatus Blautia gallistercoris, Candidatus Blautia intestinavium, Candidatus Blautia intestinigallinarum, Candidatus Blautia intestinipullorum, Candidatus Blautia merdavium, Candidatus Blautia merdigallinarum, Candidatus Blautia merdipullorum, Candidatus Blautia ornithocaccae, Candidatus Blautia pullicola, Candidatus Blautia pullistercoris, Candidatus Blautia stercoravium, Candidatus Blautia stercorigallinarum,* and *Candidatus Blautia stercoripullorum.* Preferably one or more of the following (, *Blautia glucerasea, Blautia sp. Blautia schinkii* and *Blautia wexlerae*), preferably at least *Blautia producta, Blautia glucerasea, Blautia schinkii*

In another particular embodiment, the species of the genera *Enterorhabdus* is one or more of the following species: *Enterorhabdus caecimuris, Enterorhabdus mucosicola,* and *Enterorhabdus muris*

In another particular embodiment, the species of the family *Lachnospiracceae* is one or more of the following species *Lachnospiraceae bacterium, Lachnospiraceae UCG-001* and *Lachnospiraceae UCG006*

In another particular embodiment, the species of the genera *Allobaculum* is one or more of the following species: *Allobaculum fili, Allobaculum mucilyticum* and *Allobaculum stercoricanis*

The reduction in the amount of bacterial species compared to a reference value means the total amount of *Enterorhabdus, Lachnospiracceae, Allobaculum,* and *Blautia.* The total amount can be measured by determining 16S rRNA

The method according to the preceding claim, comprising measuring the amount of bacterial species selected from the genera of *Anaeroplasma* and *Gastranaerophilales* from the first biological sample, wherein the amount of the bacterial species of said genera is increased as compared to a reference value

As used herein the term "reference value" refers to an individual with a body mass index (BMI) between 18.5 and 29.9 kg/m², preferably a BMI between 18.5 and 25 kg/m². Therefore, in the context of the present invention, the reference value means the amount of bacterial species selected from *Anaeroplasma* and *Gastranaerophilales* in a biological sample of a subject of a BMI between 18.5 and 29.9 kg/m²

The increase in the amount of bacterial species compared to a reference value means the total amount of *Anaeroplasma* and *Gastranaerophilales.* The total amount can be measured by determining 16S rRNA.

In a particular embodiment, the species of the genera *Anaeroplasma* is one or more of the following species: *Anaeroplasma abactoclasticum, Anaeroplasma bactoclasticum, Anaeroplasma intermedium,* and *Anaeroplasma varium.*

In a particular embodiment, the species of the genera *Gastranaerophilales* is one or more of the following species: *Candidatus Gastranaerophilus phascolarctosicola,* and *Candidatus Gastranaerophilus sp. (ex Termes propinquus)*

The method according to any of the preceding claims wherein the reference value is the of bacterial amount of a healthy subject, or is the bacterial amount obtained in a second biological sample from the same subject, said biological sample is obtained before the first biological sample.

A "healthy subject" being a subject with a body mass index (BMI) between 18.5 and 29.9 kg/m², preferably a BMI between 18.5 and 25 kg/m²

The method, according to any of the preceding claims, wherein the biological sample, is a sample of feaces or faecal material.

A skilled person would know how to detect the bacterial species of the genera forming part of the invention method from said biological sample using regular protocols and common general knowledge of the field, such as qPCRs of the gene 16S rRNA.

Using well-known techniques, in order to determine the full or partial 16S sequence or the sequence of any hypervariable region of the 16S sequence, genomic DNA is extracted from a biological sample, the 16S rDNA (full region or specific hypervariable regions) amplified using polymerase chain reaction (PCR), the PCR products cleaned, and nucleotide sequences delineated to determine the genetic composition of 16S gene or subdomain of the gene. If full 16S sequencing is performed, the sequencing method used may be, but is not limited to, Sanger sequencing. If one or more hypervariable regions are used, the sequencing can be, but is not limited to being performed using the Sanger method or using a next-generation sequencing method, such as an Illumina (sequencing by synthesis) method using barcoded primers allowing for multiplex reactions.

In a particular embodiment, the primer pair employed to detect the presence of microorganisms in the biological sample of a subject is
SEQ ID No 1 F (5'- TCGTCGGCAGCGTCAGATGTGTATAAGAGACAGCCTACGGGN GGCWGCAG-3') and
SEQ ID No 2 R (5'-GTCTCGTGGGCTCGGAGATGTGTATAAGAGACAGGACTACHV GGGTATCTAATCC-3')
"N" stands for any nucleotide "A", "T", "C" or "G",
"W" stands for any nucleotide "A", or "T",
"H" stands for any nucleotide "A", "T", or "C" and
"V" stands for any nucleotide "A", "C" or "G",

This primer pair detects the V3-V4 hypervariable region of the 16S rRNA gene.

In another particular embodiment, a primer pair to detect the V3-V4 hypervariable region of the 16S rRNA gene is a variant having at least 80%, preferably at least 85%, more preferably at least 90 % and even more preferably 91 % or 92 % or 93 % or 94 % or 95 % or 96 % or 97 % or 98 % or even up to 99 % identity with respect to SEQ ID No 1, and/or SEQ ID No 2. Ir is also possible that said primer pair may differ from SEQ ID No: 1 and/or SEQ ID No 2 by 1, ≤2 (i.e. equal to or less than 2), ≤3, ≤4, ≤5, ≤6 or ≤7 bases.

Therefore, in a particular embodiment of the invention, the combined preparation for uses, as described previously in the treatment and/or prevention of food addiction, the ethiology of food addiction is determined by the detection of an amount of bacterial strains selected from *Enterorhabdus, Lachnospiracceae, Allobaculum,* and *Blautia* from a first biological sample, wherein the amount of the bacterial strains is reduced as compared to a reference value.

In another particular embodiment the ethiology of food addiction is determined by the detection of an amount of bacterial strains selected from *Enterorhabdus, Lachnospiracceae, Allobaculum,* and *Blautia* from a first biological sample, wherein the amount of the bacterial strains is reduced as compared to a reference value and by the detection of the amount of bacterial strains selected from the genera of *Anaeroplasma* and *Gastranaerophilales* from the first biological sample, wherein the amount of the bacterial strains of said genera is increased as compared to a reference value.

Food addiction may have different ethiologies such as genetic alterations that produce mental disorders leading to loss of control, over eating such as that associated with bulimia nervosa and certain forms of obesity. Regarding genetic alterations: polymorphisms in the genes DRD2 and DRD4 (Ceccarini MR, Fittipaldi S, Ciccacci C, Granese E, Centofanti F, Dalla Ragione L, Bertelli M, Beccari T, Botta A.Front Nutr. 2022 Mar 14;9:838177. doi: 10.3389/fnut.2022.838177. eCollection 2022.PMID: 35369087), and 5-HT2AR and BDNF gene variants (Ceccarini MR, Tasegian A, Franzago M, Patria FF, Albi E, Codini M, Conte C, Bertelli M, Dalla Ragione L, Stuppia L, Beccari T.Am J Med Genet B Neuropsychiatr Genet. 2020 Apr;183(3):155-163. doi: 10.1002/ajmg.b.32771. Epub 2019 Nov 20 have beed disclosed to play a role in the development of food addiction.

In a particular embodiment the combined preparation of the invention is useful for use when the ethiology of food addiction is caused by genetic alterations and/or mental disorders such as the ones disclosed in the preceding paragraph.

In another particular embodiment, the combined preparation of rhamnose and lactulose has an amount of any of the prebiotics enough to augment one or more of the bacteria *Enterorhabdus, Lachnospiracceae, Allobaculum* and *Blautia* compared to the amount of bacteriaprior to the administration of the combined preparation, preferably *Blautia* species.

To quantify *Blautia* species in feaces in the biological sample, primers g-Blau-R (5'-TTGGTAAGGTTCTTCGCGTT- 3') SEQ ID No 3 and g-Blau-F (5'-GTGAAGGAAGAAGTATCTCGG- 3'), SEQ ID No 4 targeting 16S rRNA gene of said genus were used.

In another particular embodiment, the primer pair to detect *Blautia* species in a biological sample is a variant having at least 80%, preferably at least 85%, more preferably at least 90 % and even more preferably 91 % or 92 % or 93 % or 94 % or 95 % or 96 % or 97 % or 98 % or even up to 99 % identity with respect to SEQ ID No 3, and/or SEQ ID No 4. It is also possible that said primer pair may differ from SEQ ID No 3 and/or SEQ ID No 4 by 1, ≤2 (i.e. equal to or less than 2), ≤3, ≤4, or ≤5 bases.

Said genetic alterations and/or mental disorders may produce a change in the microbiota that can be used to detect food addiction in a subject. In any case, the combined preparation of the invention would augment one or more of the bacteria *Enterorhabdus, Lachnospiracceae, Allobaculum* and *Blautia* therefore ameliorating the symptoms of food addiction. This effect is independent of the genetic alterations and/or mental disorders having caused alterations in the microbiota signature.

An additional object of the invention relates to a pharmaceutical formulation comprising an effective pharmaceutical amount of the combined preparation of rhamnose and lactulose, in accordance with the first aspect of the invention, and at least one pharmaceutically acceptable excipient for use in the treatment of food addiction.

In the context of the present invention, the expression "pharmaceutical composition" refers to a formulation that has been adapted to deliver a predetermined dose of one or more useful therapeutic agents to a cell, a group of cells, an organ, or a tissue.

In the context of the present invention, "pharmaceutically acceptable excipient" means a therapeutically inactive substance that is said to be used to incorporate to the combined preparation of rhamnose and lactulose, and that is acceptable to the patient from a pharmacological/toxicological point of view and to the pharmaceutical chemist who manufactures it from a physical/chemical point of view, with respect to composition, formulation, stability, patient acceptance and bioavailability.

The pharmaceutical formulation of the invention can be suited for oral administration. At least one pharmaceutical-acceptable excipient is suitable for oral administration.

Formulations for oral administration can include powders or granules, suspensions or solutions in water or non-aqueous media, capsules, sachets, or tablets. Thickeners, flavorings, diluents, emulsifiers, dispersing aids or binders can be desirable.

Appropriate methods of administering the combined preparation, or the pharmaceutical formulation to a subject will also depend, for example, on the age and/or the physical condition of the subject and the chemical and biological properties of the compound or agent (e.g., solubility, digestibility, bioavailability, stability and toxicity). In some embodiments, a compound or an agent is administered orally, e.g., to a subject by ingestion. In some embodiments, the orally administered compound or agent is in an extended-release or slow-release formulation, or administered using a device for such slow or extended-release.

Another object of the present invention relates to a Kit or a use of a Kit to detect food addiction in a subject comprising the detection of an amount of bacterial species selected from *Enterorhabdus, Lachnospiracceae, Allobaculum,* and *Blautia* from a first biological sample, wherein the amount of the bacterial species of *Enterorhabdus, Lachnospiracceae, Allobaculum,* and *Blautia* is reduced as compared to a reference value.

Such a kit comprises the necessary reagents to detect 16S RNA genes from a biological according to the methods described herein.

In a particular embodiment, the kit comprises the primer pair SEQ ID No 1 and SEQ ID no 2 and/or a primer pair to detect the V3-V4 hypervariable region of the 16S rRNA gene is a variant having at least 80%, preferably at least 85%, more preferably at least 90 % and even more preferably 91 % or 92 % or 93 % or 94 % or 95 % or 96 % or 97 % or 98 % or even up to 99 % identity with respect to SEQ ID No 1, and/or SEQ ID No 2. It is also possible that said primer pair may differ from SEQ ID No: 1 and/or SEQ ID No 2 by 1, ≤2 (i.e. equal to or less than 2), ≤3, ≤4, ≤5, ≤6 or ≤7 bases.

In another particular embodiment, the kit is also useful for detecting Blautia species from a biological sample obtained before and/or after administering the combined preparation of the invention. For this embodiment, the kit comprises the primer pair SEQ ID No 3 and SEQ ID No. 4 and/or a primer pair to detect *Blautia* species in a biological sample being a variant having at least 80%, preferably at least 85%, more preferably at least 90 % and even more preferably 91 % or 92 % or 93 % or 94 % or 95 % or 96 % or 97 % or 98 % or even up to 99 % identity with respect to SEQ ID No 3, and/or SEQ ID No 4. It is also possible that said primer pair may differ from SEQ ID No 3 and/or SEQ ID No 4 by 1, ≤2 (i.e. equal to or less than 2), ≤3, ≤4, ≤5, ≤6 or ≤7 bases.

The kit may also contain the necessary reagents to extract RNA from a biological sample, said biological sample being, for example a feaces sample.

The kit further includes instructions and the required plastic to perform the invention method.

Components of the subject kit may be present in separate containers, or multiple components may be present in a single container. A suitable container includes a single tube (e.g., vial), one or more wells of a plate (e.g., a 96-well plate, a 384-well plate, etc.), or the like.

The instructions for using any of the kit's reagents mentioned above to perform the invention method may be recorded on a suitable recording medium. For example, the instructions may be printed on a substrate, such as paper or plastic. As such, the instructions may be present in the kits as a package insert, in labeling the kit's container or components thereof (i.e., associated with the packaging or sub-packaging), etc. In other embodiments, the instructions are presented as an electronic storage data file on a suitable computer-readable storage medium, e.g., portable flash drive, DVD, CD-ROM, diskette, etc. In yet other embodiments, the actual instructions are not present in the kit, but means for obtaining the instructions from a remote source, e.g. via the internet, are provided. An example of this embodiment is a kit that includes a web address where the instructions can be viewed and/or from which the instructions can be downloaded. As with the instructions, the means for obtaining the instructions is recorded on a suitable substrate.

When referring to sequence identity between two sequences, their sequences are compared and the amount of identical nucleotides between the two sequences is determined. For example, a first nucleic acid sequence having at least 80% nucleic acid sequence identity with a second sequence requires that, following the alignment of the first nucleic acid sequence with the second sequence, at least 80% of the nucleotides in the first nucleic acid sequence are identical to the corresponding nucleotides in the second sequence.

Typically, the sequence comparison is carried out over the length of the reference sequence. For example, if the user wished to determine whether a given ("test") sequence is 95% identical to SEQ ID No 1, SEQ ID No 1 would be the reference sequence. To assess whether a sequence is at least 95% identical to SEQ ID No 1 (an example of a reference sequence), the skilled person would carry out an alignment over the length of SEQ ID No 1, and identify how many positions in the test sequence were identical to those of SEQ ID No 1. If at least 95% of the positions are identical, the test sequence is at least 95% identical to SEQ ID No 1. If the sequence is shorter than SEQ ID No 1, the gaps or missing positions should be considered to be non-identical positions.

Sequences are typically aligned for identity calculations using a mathematical algorithm, such as the algorithm of Karlin and Altschul (Proc. Natl. Acad. Sci. USA 87 (1990): 2264 2268), modified as in Karlin and Altschul (Proc. Natl. Acad. Sci. USA 90 (1993): 5873 5877). Such an algorithm is incorporated into the XBLAST programs of Altschul et al. (J. Mol. Biol. 215 (1990): 403 410). To obtain gapped alignments, Gapped BLAST can be utilized as described in Altschul et al. (Nucleic Acids Res. 25 (1997): 3389 3402). When utilizing BLAST and Gapped BLAST programs, the default parameters of the respective programs can be used.

The BLAST algorithm involves first identifying high scoring sequence pairs (HSPs) by identifying short words of length W in the query sequence that either match or satisfy some positive-valued threshold score T when aligned with a word of the same length in a database sequence. T is referred to as the neighbourhood word score threshold (Altschul et al, supra). These initial neighbourhood word hits act as seeds for initiating searches to find HSPs containing them. The word hits are extended in both directions along each sequence for as far as the cumulative alignment score can be increased. Extensions for the word hits in each direction are halted when the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLAST program uses as defaults a word length (W) of 11, the BLOSUM62 scoring matrix (see Henikoff and Henikoff (1992) Proc. Natl. Acad. Sci. USA 89: 10915-10919) alignments (B) of 50, expectation (E) of 10, M=5, N=4, and a comparison of both strands.

The BLAST algorithm performs a statistical analysis of the similarity between two sequences; see e.g., Karlin and Altschul (1993) Proc. Natl. Acad. Sci. USA 90: 5873-5787. One similarity measure provided by the BLAST algorithm is the smallest sum probability (P(N)), which indicates the probability by which a match between two polynucleotide or amino acid sequences would occur by chance. For example, a sequence is considered similar to another sequence if the smallest sum probability in comparison of the first sequence to the second sequence is less than about 1, preferably less than about 0.1, more preferably less than 0.01, and most preferably less than about 0.001. Alternatively, the UWGCG Package provides the BESTFIT program, which can be used to calculate identity (for example, used on its default settings) (Devereux et al (1984) Nucleic Acids Research 12, 387-395).

Each of the terms "comprising," "consisting essentially of," and "consisting of" may be replaced with either of the other two terms. The term "a" or "an" can refer to one of or a plurality of the elements it modifies (e.g., "a reagent" can mean one or more reagents) unless it is contextually clear either one of the elements or more than one of the elements is described. The term "about" as used herein refers to a value within 10% of the underlying parameter (i.e., plus or minus 10%; e.g., a weight of "about 100 grams" can include a weight between 90 grams and 110 grams). Use of the term "about" at the beginning of a listing of values modifies each of the values (e.g., "about 1, 2 and 3" refers to "about 1, about 2 and about 3"). When a listing of values is described the listing includes all intermediate values and all fractional values thereof (e.g., the listing of values "80%, 85% or 90%" includes the intermediate value 86% and the fractional value 86.4%). When a listing of values is followed by the term "or more," the term "or more" applies to each of the values listed (e.g., the listing of "80%, 90%, 95%, or more" or "80%, 90%, 95% or more" or "80%, 90%, or 95% or more" refers to "80% or more, 90% or more, or 95% or more"). When a listing of values is described, the listing includes all ranges between any two of the values listed (e.g., the listing of "80%, 90% or 95%" includes ranges of "80% to 90%", "80% to 95%" and "90% to 95%"). Certain implementations of the technology are set forth in the examples below.

### Brief description of the figures

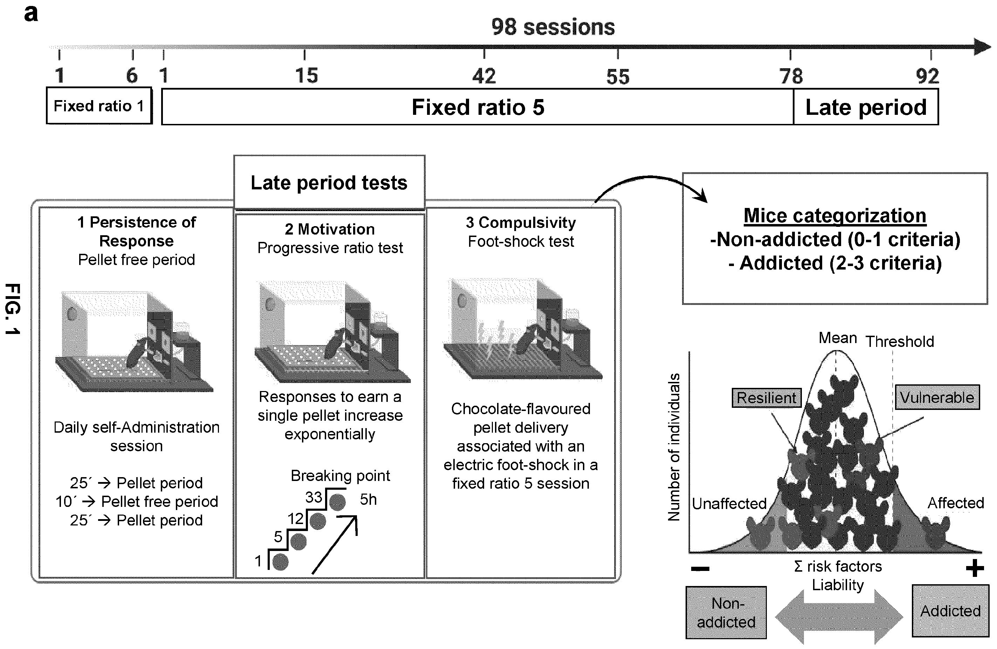

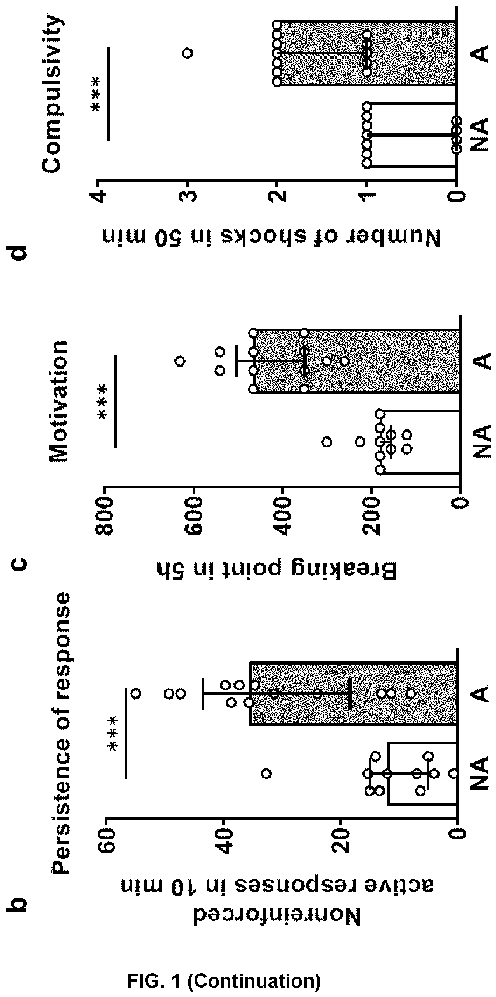

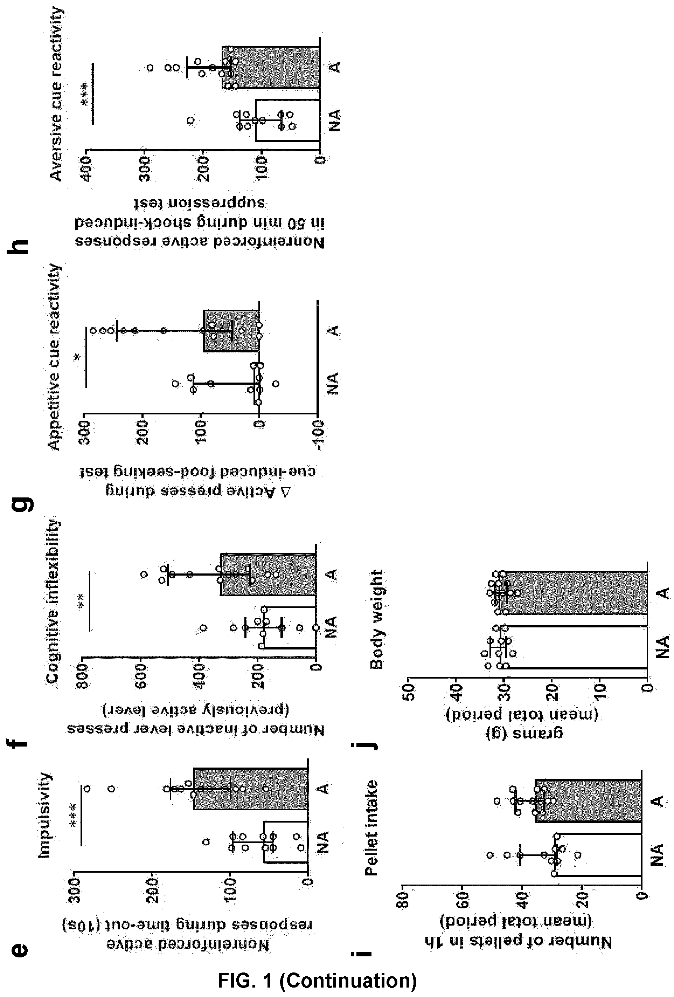

**Fig. 1****. Characterization of extreme subpopulations of addicted and nonaddicted mice. a** Timeline of the procedure of operant behavior mouse model. Mice were trained during the first six days in operant behavior sessions of one hour at a fixed ratio (FR) 1 schedule of reinforcement, followed by 92 daily sessions of FR5. The addiction-like criteria (persistence of response, motivation, and compulsivity) were evaluated in the late period to categorize mice into addicted and nonaddicted. (**b-d**). Behavioral tests for the three addiction-like criteria in the late period (individual values with the interquartile range) in the addicted and nonaddicted groups. **b** persistence of response (t-test,*** P<0.001). **c** Motivation (Mann-Whitney's U, *** P<0.001). **d** Compulsivity (Mann-Whitney's U,*** P<0.001). (e-h) Behavioral tests for the four phenotypic traits associated with vulnerability to food addiction in the late period (individual values with the interquartile range). **e** Impulsivity (t-test, *** P<0.001). **f** Cognitive inflexibility (t-test,** P<0.01). g Appetitive cue reactivity (U Mann-Whitney, *P<0.05). **h** Aversive cue reactivity (t-test,***P<0.00).

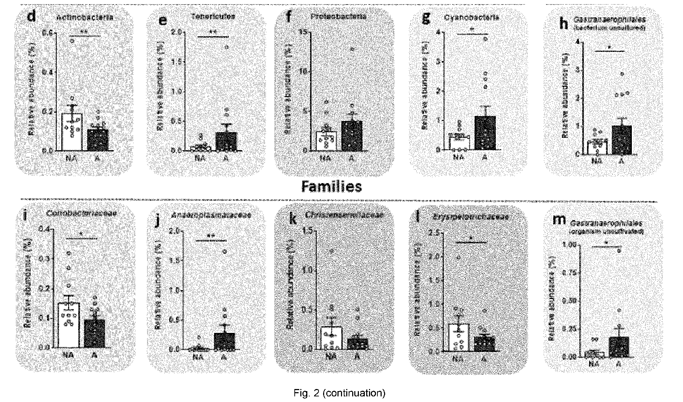

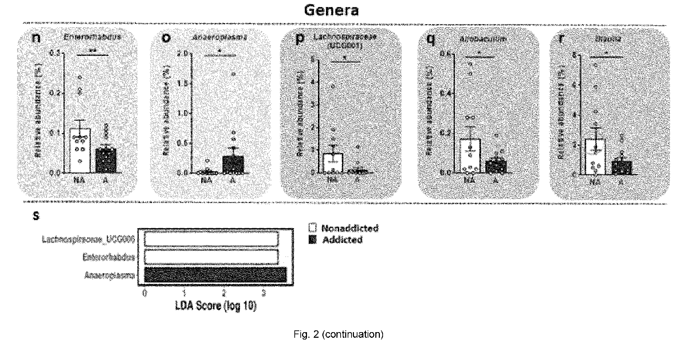

**Fig. 2****. Gut microbiota profile of vulnerability to addiction in mice. a Pie chart at the** phylum level shows a high proportion of the phyla Firmicutes and Bacteroidetes, reaching almost 90% of the relative abundance in both groups of addicted and nonaddicted mice. **b-c** Results of Chao1 and Shannon diversity indexes of addicted a and nonaddicted (NA) mice. **d-r** Differences between addicted and nonaddicted mice after a long operant training protocol were observed in the relative abundances at **d-g** phylum, **h-m** family, and **n-r** genus level. A color shadow behind each graph is depicted according to the color of the phyla they belong. **s** Discriminant analysis effect size method (LEfSe) comparing addicted and nonaddicted groups. Data are expressed as mean ± S.E.M. DESeq2 test, *P<0.05, **P<0.01, (n=11 nonaddicted ("NA") mice, n=13 addicted ("A") mice).

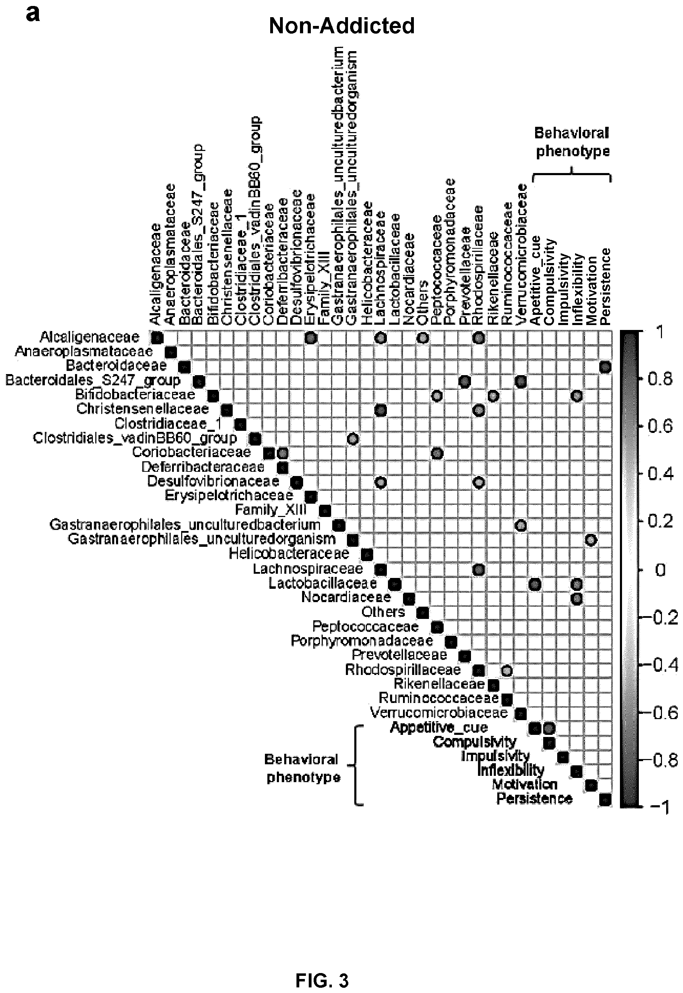

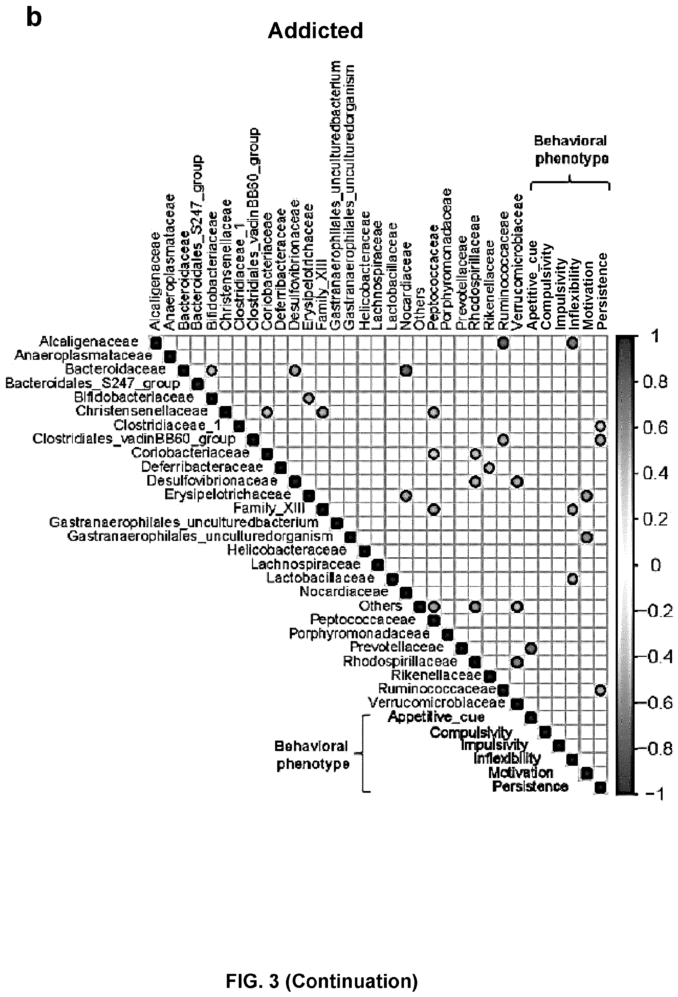

**Fig. 3****. Gut microbiota correlates with food addiction features, a** Linear discriminant analysis (LDA) scores of taxa significantly discriminant between addicted and nonaddicted mice (LDA scores > 2 and significance of P<0.05 as determined by Wilcoxon's signed-rank test). **b** Corrplot showing significant (P<0,05) Spearman correlations coefficients between microbiota relative abundances at the family level and phenotypic compulsivity trials in addicted and nonaddicted mice after a long operant training protocol. (n = 11 for non-addicted mice and n = 13 for addicted mice).

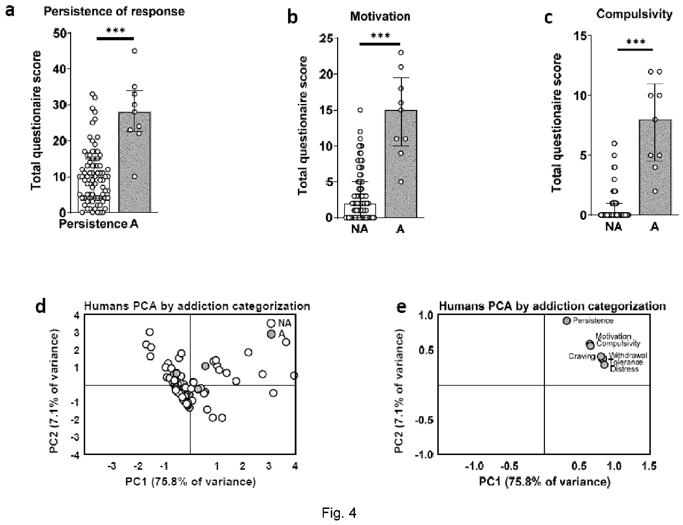

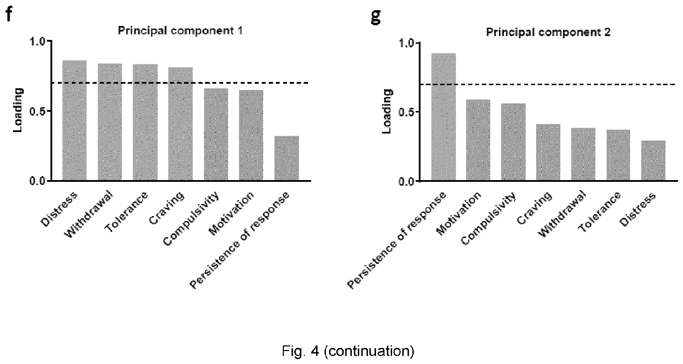

**Fig. 4****. Gut microbiota signatures in humans. a-c** Results of the three addict-like criteria. **a** Persistence of response, **b** motivation (MannWhitney's U, *** P<0.001), and **c** compulsivity (MannWhitney's U, *** P<0.001) (median with the interquartile range), comparing "NA" and "A" participants. n=28 for human participants (n=25 nonaddicted ("NA") individuals, n=3 addicted ("A") individuals). (**d-g**) Principal component analysis (PCA) of the three addiction criteria and the four phenotypic traits in humans. **d** Mice subjects clustered by addicted or nonaddicted classification on the space yielded by two components of the PCA that account for the maximum data variance. **e** Criteria belonging to each component, principal component (PC) 1 (60.5%) and PC2 (19.2%). **f, g** The order of factor loading of the different variables in the PC1 and PC2 is represented. The dashed horizontal line marked loadings >0.7, mainly contributing to the component.

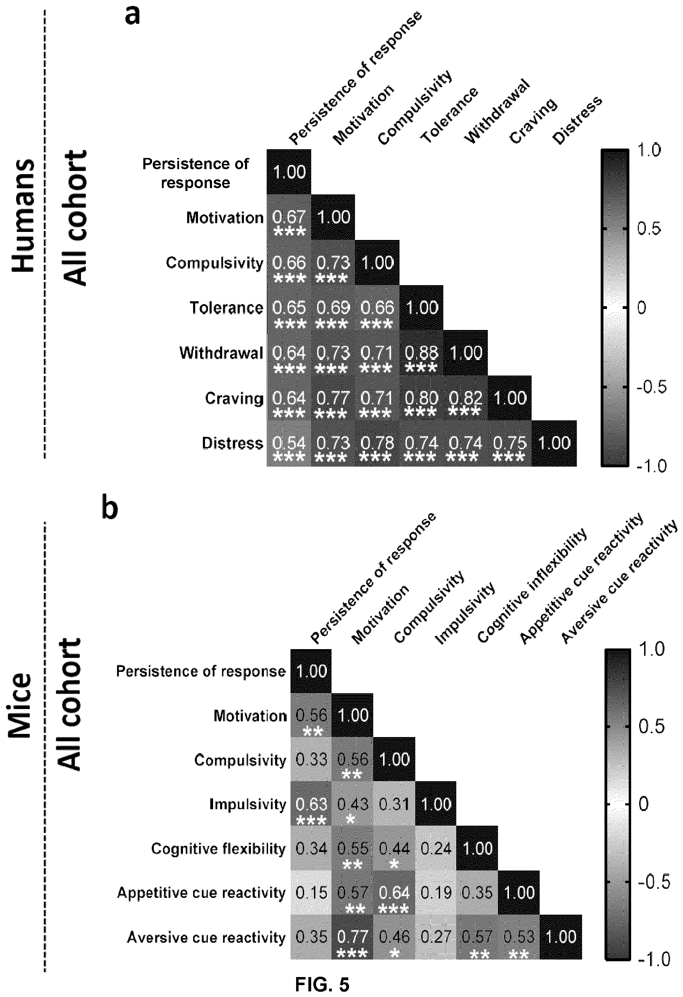

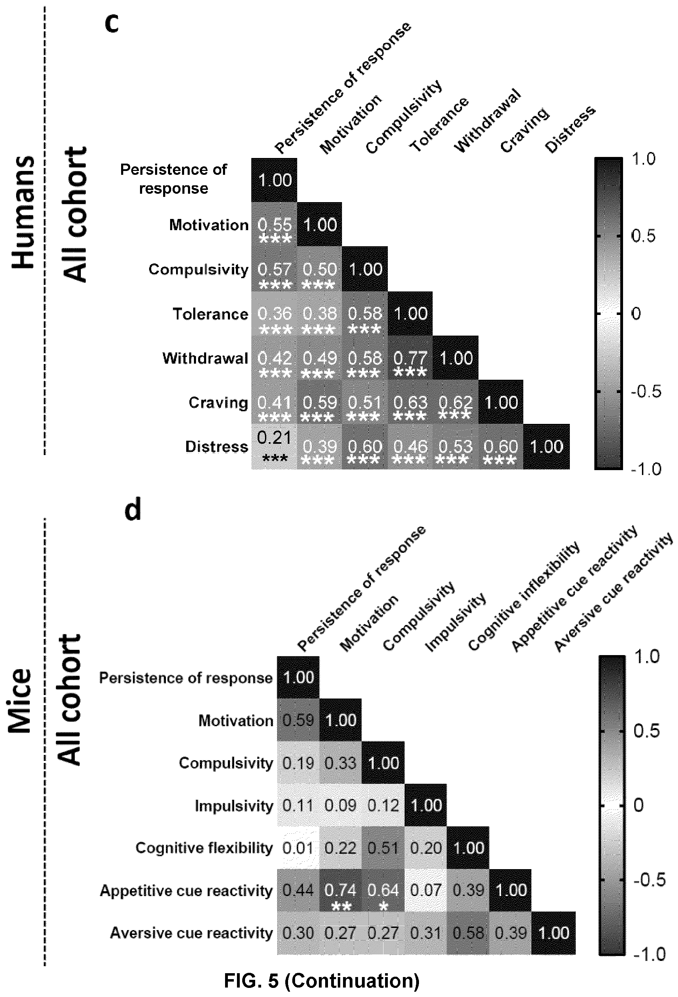

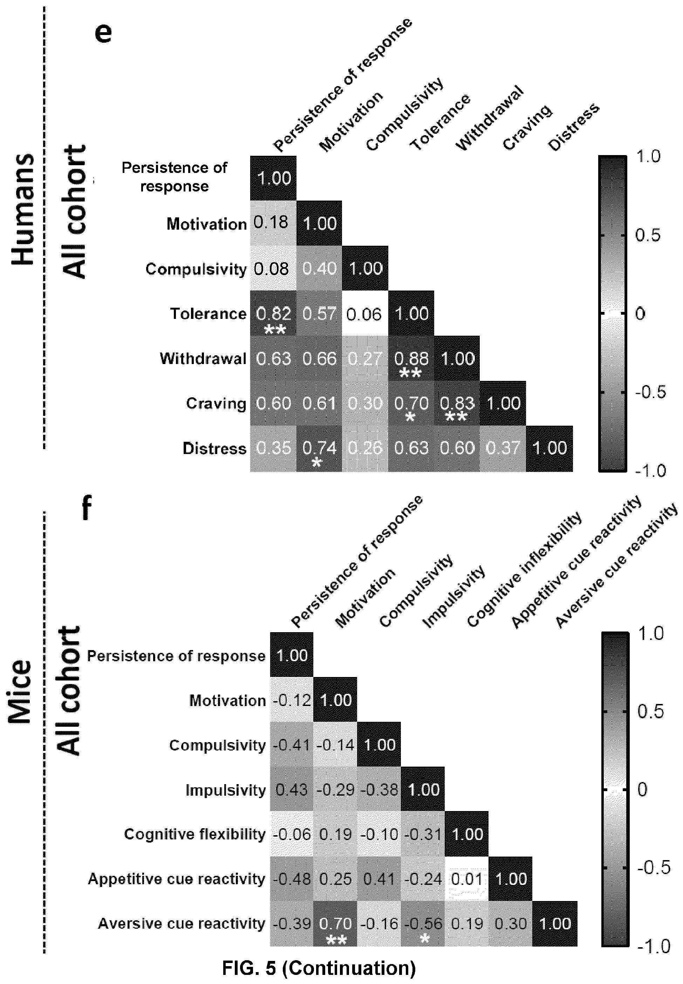

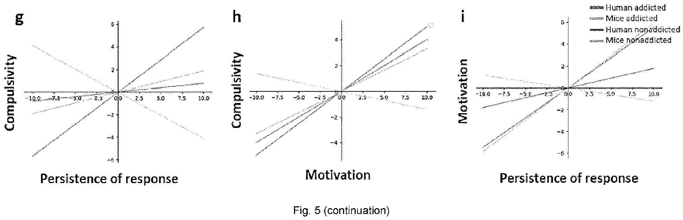

**Fig 5****. Correlation heat map matrix of the three addiction criteria and other four phenotypic traits.** Heatmap of **a** total human subjects, **c** nonaddicted human subjects, **e** addicted human subjects, **b** total mice population, **d** nonaddicted mice population, **f** addicted mice population. Pearson's correlations, *P<0.05, **P<0.01, ***P<0.001 (n=25 nonaddicted ("NA") mice, n=3 addicted ("A") mice). **g-i** Correlational analyses between addiction criteria of persistence of response, motivation and compulsivity in addicted and nonaddicted mice compared to addicted and nonaddicted individuals belonging to the human cohort were analyzed together. **g** In the correlation between compulsivity and persistence of response, a strong negative correlation for addicted individuals (both human and mice) and a moderate positive correlation for nonaddicted individuals were described. **h** In the correlation between compulsivity and motivation, a mild negative correlation for addicted individuals (mice) was found, while positive correlations for nonaddicted individuals (mice and humans) and addicted humans were described. i In the correlation between motivation and persistence of response, a strong negative correlation for addicted individuals (humans) and mild for mice was found together with a moderate positive correlation for nonaddicted individuals (mice and humans) with a similar slope.

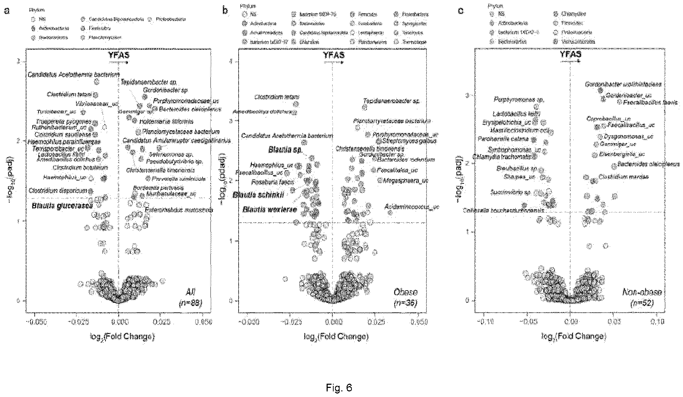

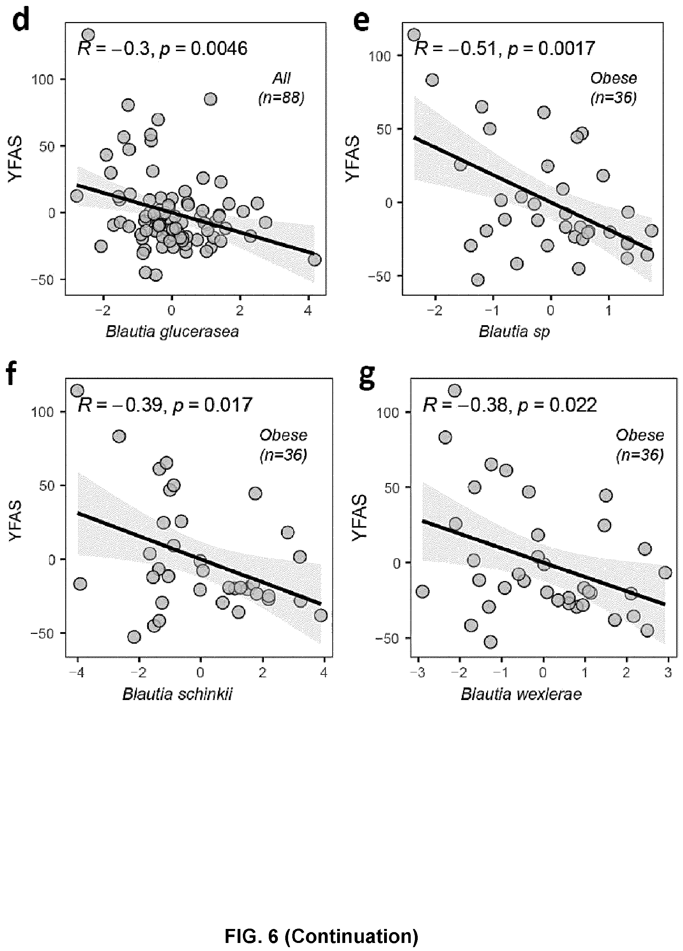

**Fig 6****. Volcano plot of bacterial abundance.** A volcano plot representing the differential bacterial abundance (pFDR < 0.05) using ANCOM-BC controlling for age, BMI, and sex in humans (n=28). Fold change (FC) associated with a unit change in and Benjamini-Hochberg p values (pFDR) are plotted for each taxon. Significantly different taxa are coloured according to the phylum.

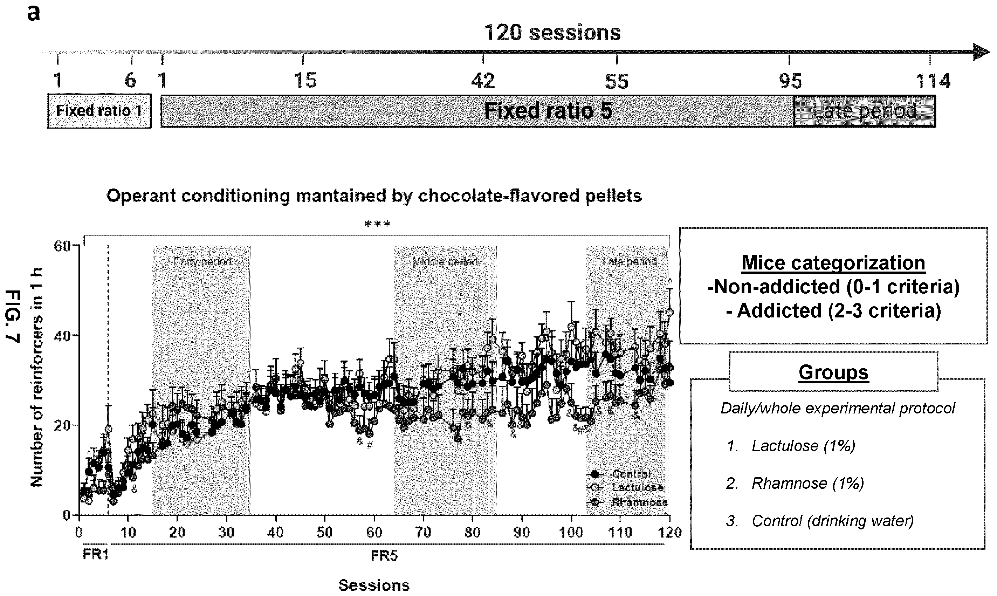

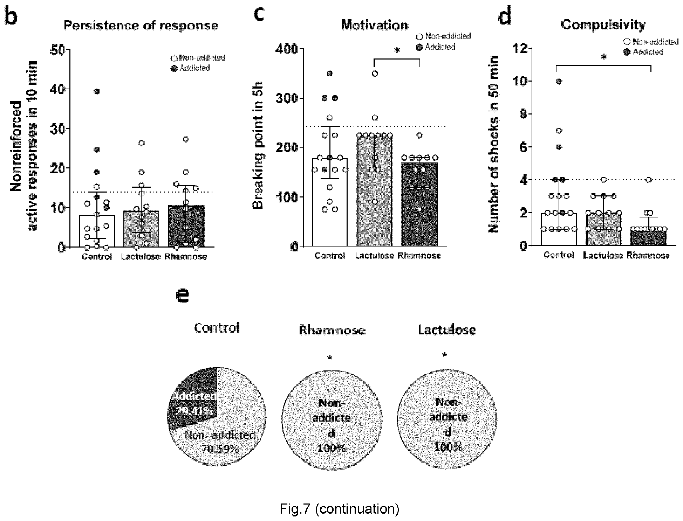

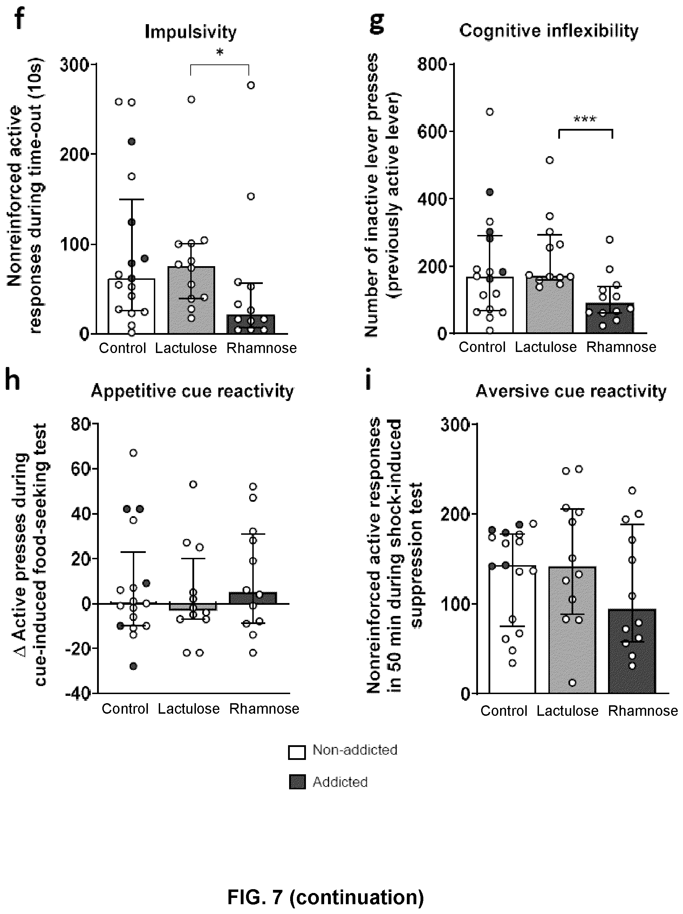

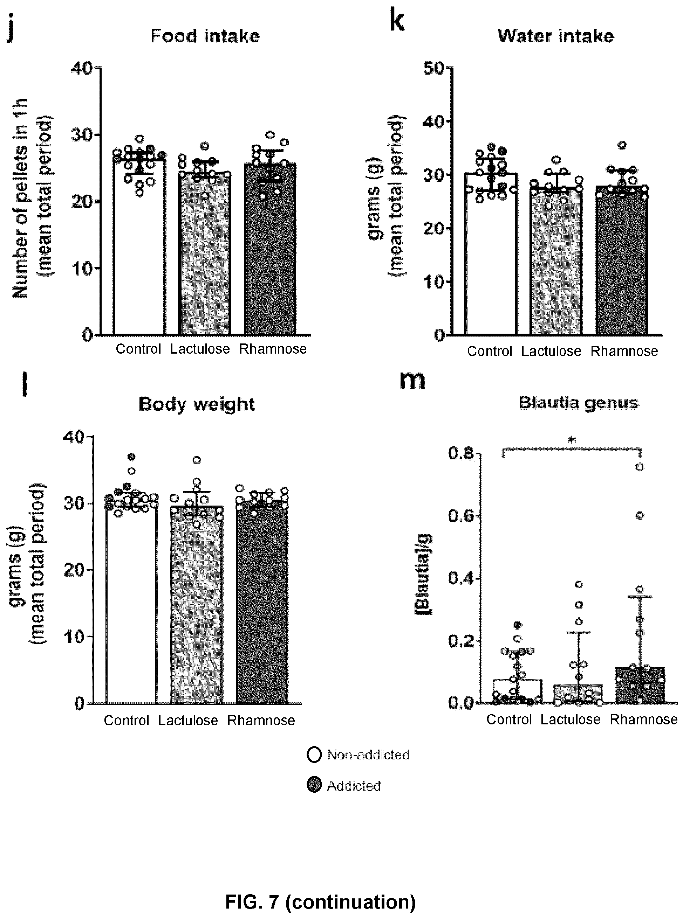

**Fig. 7****. Characterization of extreme subpopulations of addicted and nonaddicted mice in the experiment with prebiotics. a** Timeline of the procedure of operant behavior mouse model. Mice were trained during the first six days in operant behavior sessions of one hour at a fixed ratio (FR) 1 schedule of reinforcement, followed by 120 daily sessions of FR5. The addiction-like criteria (persistence of response, motivation, and compulsivity) were evaluated in the late period (95-112) to categorize mice into addicted and nonaddicted. Mice received the prebiotic lactulose, rhamnose, or control in drinkable water during the whole experimental sequence. Number of reinforcers during 1 h of operant training sessions maintained by chocolate-flavored pellets in the three groups (mean ± S.E.M, repeated measures ANOVA, session x treatment effect ***P<0.001, post-hoc Newman-Keuls, ^P<0.05 control vs. lactulose, &P<0.05 lactulose vs. rhamnose). (**b-d**) Behavioral tests for the three addiction-like criteria in the late period (individual values with the interquartile range) in the addicted and nonaddicted groups. **b** persistence of response. **c** Motivation (MannWhitney's U, *** P<0.05). **d** Compulsivity (Mann-Whitney's U,* P<0.05). **e** Percentage of mice classified into addicted and nonaddicted in the groups of control lactulose and rhamnose respectively. (**f-i**) Behavioral tests for the four phenotypic traits associated with vulnerability to food addiction in the late period (individual values with the interquartile range). **f** Impulsivity. **g** Cognitive inflexibility (MannWhitney's U, * P<0.05, ** P<0.01). **h** Appetitive cue reactivity. **i** Aversive cue reactivity. **j** Pellets intake, **k** water intake and l body weight. m [*Blautia*]/*g* in mice feces determined by pPCR. The sample size of mice in the lactulose, rhamnose and control groups was n=12 per group (total n=36).

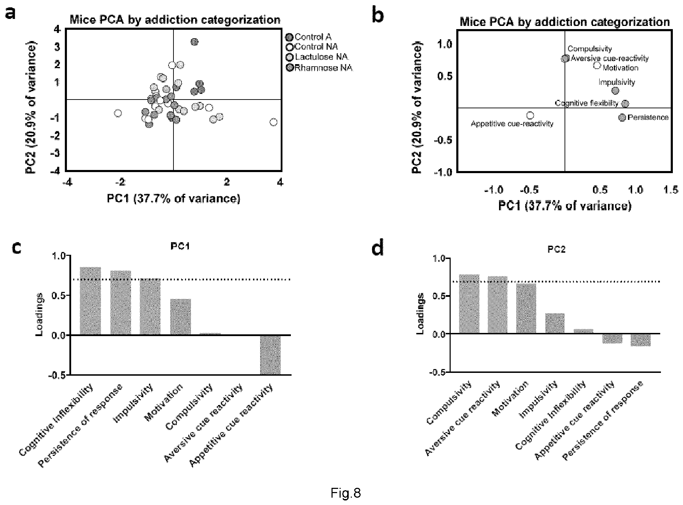

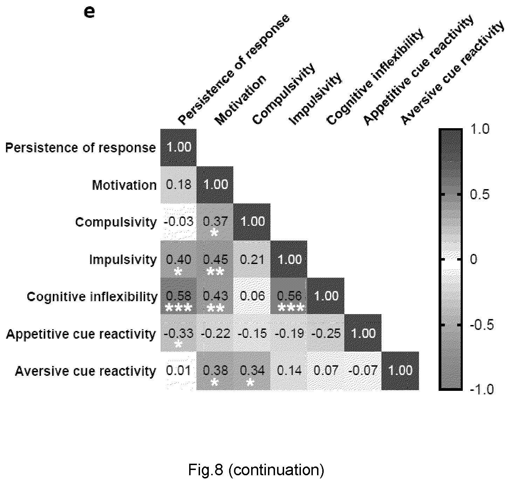

**Fig. 8****. Principal component analysis revealed differential patterns of behavioral factor loadings in food addiction-like behavior in mice treated with prebiotics. a** Mice subjects clustered by addicted or nonaddicted classification on the space yielded by two components of the PCA that account for the maximum data variance (n=5 control addicted mice, n=7 control nonaddicted mice, n=12 lactulose nonaddicted mice, n=12 rhamnose nonaddicted mice). **b** Criteria belonging to each component, principal component (PC) 1 (28.1%) and PC2 (21.1%). **c,d** The order of factor loading of the different variables in the PC1 and PC2 is represented. The dashed horizontal line marked loadings >0.7, mainly contributing to the component. e Heatmap correlation matrix of the three addiction criteria and the four phenotypic traits. Colors correspond to the magnitude of Pearson correlations between each pair of variables and range from -1 (red) to +1 (blue). Significant Pearson's correlations are detailed with symbols, *P<0.05, **P<0.01, ***P<0.001, n=36 mice.

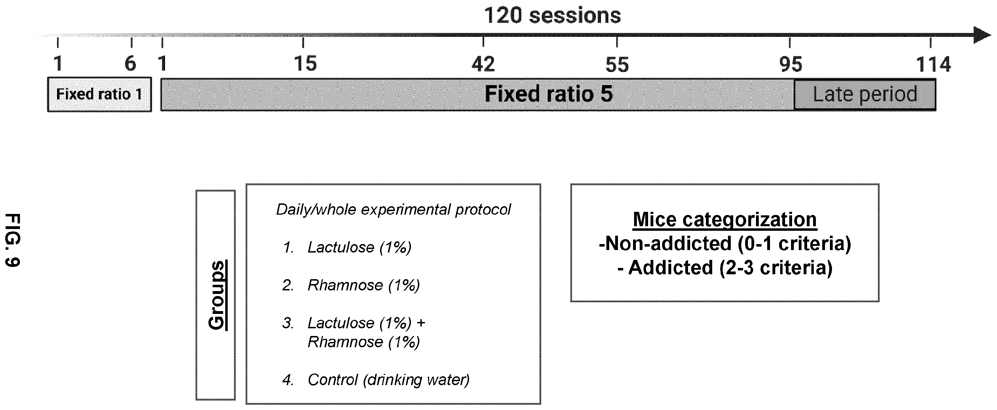

**Fig. 9****. Characterization of extreme subpopulations of addicted and nonaddicted mice in an experiment with the combined preparation of the invention. a** Timeline of the procedure of operant behavior mouse model. Mice were trained during the first six days in operant behavior sessions of one hour at a fixed ratio (FR) 1 schedule of reinforcement, followed by 120 daily sessions of FR5. The addiction-like criteria (persistence of response, motivation, and compulsivity) were evaluated in the late period (95-114) to categorize mice into addicted and nonaddicted. Mice received the prebiotic lactulose, rhamnose, the combined preparation of the invention or control in drinkable water during the whole experimental sequence. Number of reinforcers during 1 h of operant training sessions maintained by chocolate-flavored pellets in the four groups

### EXAMPLES

### Methods

### Animals

A previous experiment to evaluate miRNA signatures associated with vulnerability to food addiction in a large cohort of male JAX^{™} C57BL/6J mice aged eight weeks (Charles River, Lyon, France) (Garcia-Blanco A, Domingo-Rodriguez L, Cabana-Dominguez J, et al. miRNA signatures associated with vulnerability to food addiction in mice and humans. J Clin Invest. 2022;132(10):e156281. doi:10.1172/JCI156281). From this cohort, subgroups of extreme nonaddicted (n=11) and addicted (n=13) mice were selected to evaluate the microbiota signature associated with vulnerability and resilience to develop food addiction. Mice were housed and maintained individually in controlled laboratory conditions (21 ± 1°C and 55 ± 10% humidity), with food and water available *ad libitum* during the entire experiment. Mice were tested during the dark phase of a reverse light cycle (lights off at 8.00 a.m. and on at 8.00 p.m.).

### Mouse food addiction protocol

Mice were trained to obtain chocolate-flavored pellets (n=59) in operant boxes during 98 sessions in 1 h-daily self-administration sessions. The operant boxes and daily self-administration protocol of food addiction were accurately described in previous publications (Martín-García E, Domingo-Rodriguez L, Maldonado R. An Operant Conditioning Model Combined with a Chemogenetic Approach to Study the Neurobiology of Food Addiction in Mice. Bio-Protocol 2020;10:1-23. doi:10.21769/bioprotoc.3777; Domingo-Rodriguez L, Ruiz de Azua I, Dominguez E, et al. A specific prelimbic-nucleus accumbens pathway controls resilience versus vulnerability to food addiction. Nat Commun 2020;11:1-16. doi:10.1038/s41467-020-14458-y). Mice were under fixed-ratio 1 (FR1) during six sessions, followed by 92 sessions under FR5. The food addiction-like behavior was evaluated during this operant conditioning maintained by food at a late period (82-92 sessions) to classify mice into the addictive phenotype considering three food addiction-like criteria (1) persistence of response, (2) motivation, and (3) compulsivity. These criteria summarized the main features of the human food addiction diagnosis through the Yale Food Addiction Scale (YFAS 2.0) based on the 5^{th} edition of the diagnostic and statistical manual of mental disorders (DSM-5) for substance use disorders. The three behavioral tests performed to evaluate these three addiction-like criteria (responding during the pellet-free period for the persistence of response, progressive ratio schedule for motivation, and shock-associated unconditioned stimulus for compulsivity) were performed as previously described (Mancino S, Burokas A, Gutiérrez-Cuesta J, et al. Epigenetic and Proteomic Expression Changes Promoted by Eating Addictive-Like Behavior. Neuropsychopharmacology 2015;40:2788-800. doi:10.1038/npp.2015.129). At the end of the late period, animals were categorized into two subpopulations, food-addicted or nonaddicted, depending on the number of addiction-like criteria achieved. Mice with two or three addiction-like criteria were considered addicted, and mice with 0 or 1 addiction-like criteria were considered nonaddicted.

Considering the individual score in each addiction-like criteria, extreme phenotypes were selected of addicted and nonaddicted mice to evaluate changes in the gut microbiota underlying the loss of control toward palatable food, and the caecum was extracted immediately after the last FR5 session for these studies.

Additionally, four additional phenotypic traits were also evaluated as factors of vulnerability, impulsivity, cognitive inflexibility, appetitive and aversive cue-reactivity.
*Impulsivity,* the inability to stop a response once it is initiated, was measured as the number of non-reinforced active responses during the time-out period (10 s) after each pellet delivery. This impulsivity-like behavior was delimited to the three consecutive days before the progressive ratio.
*Cognitive inflexibility* is the incapacity to shift responding to stimuli that have previously predicted the availability of reward. It is measured by the ability to modify the operant behavior when the active and the inactive levers were reversed in a single training session without previous learning. The errors are the number of active-reversed responses (previous inactive lever in a typical training session) performed in 1 h.
*Appetitive cue-reactivity*: The cue-induced food-seeking test, which consisted of a 90-min session, assessed the conditioning to an appetitive stimulus (cue-light). In the first 60 min, all active and inactive lever presses were recorded but produced no consequences. In the next 30 min, the cue light associated with pellet delivery during a typical operant training session was illuminated with no contingent pellet reinforcement. To signal the change in the schedule, the cue light was presented twice non-contingently and for 4 s.
*Aversive cue-reactivity*: To study the conditioning to an aversive stimulus (grid floor), non-reinforced active responses during the following session after the shock test were measured. Mice were placed in the operant box for one hour with the same grid floor used during the shock test. However, during this session, pressing the active lever had no consequences: no shock, no chocolate-flavored pellets, and no cue light.

### Food pellets

Chocolate-flavored pellets (20 mg, highly isocaloric pellet, TestDiet, Richmond, IN, US) were delivered after pressing the active lever during the operant conditioning sessions. Chocolate-flavored pellets contain 20.6% protein, 12.7% fat, and 66.7% carbohydrate, with a caloric value of 3.44 kcal/g. Even though chocolate-flavored pellets included slight differences in their composition with an addition of chocolate flavor (2% pure unsweetened cocoa) and a higher proportion of sucrose content (50.1%) compared to standard pellets (8.3%), chocolate-flavored pellets presented a similar percentage of carbohydrates and caloric value as standard pellets (65.5% carbohydrate and caloric value of 3.30 kcal/g).

### Gut microbiota profile of vulnerability to addiction in mice

DNA was extracted for the caecal content using the QIAamp DNA stool kit (Qiagen, Sussex, UK) following the manufacturer's instructions, with the addition of an initial bead-beating step. Extracted DNA was kept frozen at -20°C until analysis.

For 16S rRNA Gene Sequence-based microbiota analysis, the V3-V4 hypervariable region of the 16S rRNA gene was amplified by PCR reaction on the template DNA using the primer pair [F (5'-TCGTCGGCAGCGTCAGATGTGTATAAGAGACAGCCTACGGGNGGCWGCAG-3') SEQ ID No 1 and R (5'-GTCTCGTGGGCTCGGAGATGTGTATAAGAGACAGGACTACHVGGGTATCTAATCC-3')] SEQ ID No 2 according to the 16S Metagenomic Sequencing preparation protocol for Illumina MiSeq. Following WIPO ST. 26:
"N" stands for any nucleotide "A", "T", "C" or "G",
"W" stands for any nucleotide "A", or "T",
"H" stands for any nucleotide "A", "T", or "C" and
"V" stands for any nucleotide "A", "C" or "G",
PCR amplicons were purified using the Agencourt AMPure XP system (Beckman Coulter Genomics, Takeley, UK) following the Illumina 16S Metagenomic Sequencing Library preparation guide (Illumina) and library was prepared. Briefly, dual indices and Illumina sequencing adapters were attached using the Nextera XT Index Kit (Illumina, San Diego, CA), and a second clean-up step using the Agencourt AMPure XP system was performed. Library quantification, normalization, pooling, and denaturation were performed. Samples were sequenced on the MiSeq sequencing platform at Teagasc, using a 2 × 300 cycle Kit, following standard Illumina sequencing protocols.

Both SEQ ID No 1 and SEQ ID No 2 were used to detect all the bacterial species of the microbiota signature: *Enterorhabdus, Lachnospiracceae, Allobaculum, Blautia, Anaeroplasma* and *Gastranaerophilales.*

The bioinformatics sequence and statistical analysis were performed with paired-end reads using fast length adjustment of short reads to improve genome assemblies (FLASH) and the Quantitative Insights into Microbial Ecology (QIIME) suite of tools, v1.9.0, for further processing of paired-end reads, including quality filtering based on a quality score of > 25 and removal of mismatched barcodes and sequences below length thresholds. Deionization, chimera detection, and operational taxonomic unit (OTUs; 97% identity) grouping were performed in QIIME using USEARCH v7. Taxonomic ranks were assigned (97% similar identity) by alignment of OTUs using python nearest alignment space termination (PyNAST) to the SILVA SSURef database release 123.

### Yale Food Addiction Scale in humans

The YFAS is a 35-item questionnaire that assesses addictive-like eating according to the diagnostic criteria for drug dependence in the DSM-IV (Gearhardt AN, Corbin WR, Brownell KD. Preliminary validation of the Yale Food Addiction Scale. Appetite 2009;52:430-6. doi:10.1016/j.appet.2008.12.003; Schulte EM, Gearhardt AN. Development of the Modified Yale Food Addiction Scale Version 2.0.

Eur Eat Disord Rev 2017;25:302-8. doi:10.1002/erv.2515). The YFAS has been shown to have sufficient psychometric properties (original validation study Cronbach alpha=0.86). The tool includes two scoring options, a symptom score based on the DSM-IV criteria for drug dependence, a diagnosis of food addiction, and clinical impairment or distress.

In this study, we used the Spanish YFAS 2.0, a 35-item self-report instrument that measures the presence or absence of food addiction symptoms based on the eleven criteria for Substance-Related and Addictive Disorders (SRAD) in the DSM-5 adapted to the context of highly processed food (Gearhardt AN, Corbin WR, Brownell KD. Development of the Yale Food Addiction Scale Version 2.0. Psychol Addict Behav 2016;30:113-21. doi:10.1037/adb0000136). Each YFAS 2.0 question had eight frequency responses (0="never", 7="every day"), and depending on this, the question threshold was met or not met. As specific YFAS 2.0 questions represented one DSM-5 SRAD criterion, if the sum of questions under one addiction criterion was ≥1, then the addiction criterion was met and scored 1. If the sum was 0, the addiction criterion was not met and scored 0. Finally, it was counted the number of addiction criteria met, and reaching two addiction criteria was sufficient to be considered food addiction if clinical significance impairment or distress were reported. Certain YFAS 2.0 questions represent a specific DSM-5 addiction criterion, and in order to mimic the food addiction criteria evaluated in our mouse model, specific DSM-5 criteria were grouped into the same three addiction-like criteria, (1) persistence of response, (2) motivation, and (3) compulsivity. Each addiction-like criterion was denoted by exact YFAS 2.0 questions as follows:
- Persistence of response, YFAS 2.0 questions 1, 2, 3, 4, 25, 31, and 32.
- Motivation, YFAS 2.0 questions 5, 6, 7, 8, 10, 18, and 20.
- Compulsivity, YFAS 2.0 questions 22 and 23.

In this study, the total question score for each addiction-like criterion in human graphs was the sum of the frequency mark obtained in each YFAS 2.0 question. The YFAS 2.0 was translated into Spanish according to the International Test Commission Guidelines for Translating and Adapting Tests by two bilingual clinical psychologists experts in eating disorders. The validated Spanish version of YFAS 2.0 showed good psychometric properties with internal consistency, accuracy, and discriminative and convergent validity with other eating-related constructs. All models in humans were adjusted for age, body mass index (BMI), and gender.

### Gut microbiota signatures in humans

The gut microbiota signatures in humans were performed using the IRONMET-CGM (n=28) cohort. The Glucose, Brain, and Microbiota study (IRONMET+CGM, ClinicalTrials.gov: https://clinicaltrials.gov/ct2/show/NCT03889132) is a case-control study conducted at the Endocrinology Department of Dr. Josep Trueta University Hospital. The recruitment of subjects started in March 2019 and finished in March 2022. The cohort includes a subset of 15 middle-aged patients [52.6 years [47.6-56.1]] with obesity (BMI ≥30 kg/m2) and 13 age-matched and sex-matched subjects without obesity (BMI 18.5-<30 kg/m2) that have measurements of the YFAS. Exclusion criteria included: type 2 diabetes mellitus, chronic inflammatory systemic diseases, acute or chronic infections in the previous month; use of antibiotic, antifungal, antiviral or treatment with proton pump inhibitors; severe disorders of eating behavior or major psychiatric antecedents; neurological diseases, history of trauma or injured brain, language disorders and excessive alcohol intake (≥20g OH/day in women or 40g OH/day in men). Notably, given the important confounding effects of alcohol intake, all subjects included here had an intake of less than 20g/day.

### Extraction of fecal genomic DNA and whole-genome shotgun sequencing in humans

Total DNA was extracted from frozen human stools using the QIAamp DNA mini stool kit (QIAGEN, Courtaboeuf, France). Quantification of DNA was performed with a Qubit 3.0 fluorometer (Thermo Fisher Scientific, Carlsbad, CA, USA), and 1 ng of each sample (0.2 ng/µl) was used for shotgun library preparation for high-throughput sequencing, using the Nextera DNA Flex Library Prep kit (Illumina, Inc., San Diego, CA, USA) according to the manufacturer's protocol. Sequencing was carried out on a NextSeq 500 sequencing system (Illumina) with 2 X 150-bp paired-end chemistry at the facilities of the Sequencing and Bioinformatic Service of the FISABIO (Valencia, Spain). For the taxonomic analysis of the microbiota present in the samples, FASTQ output files were first preprocessed using *fastp,* a FASTQ data pre-processing tool for quality control, trimming of adapters, and quality filtering. Clean reads were mapped against the Homo sapiens genome database (GRCh38.p13) using *Bowtie2* to remove reads from human origin. Unmapped reads were run using the *SqueezeMeta v1.3.1* using the co-assembly mode to pool all samples in a single assembly. Contigs assembly was carried out with *megahit* the mapping of reads in contigs was performed with *Bowtie2. Prodigal* was used for ORFs prediction, and *Diamon* for ORF search and alignment against the GenBank nr database for taxonomic assignment.

### Functional validation with prebiotics treatment in the mouse food addiction protocol

Mice (n=36) were trained in the same experimental conditions previously described to obtain chocolate flavored pellets during 120 sessions of a 1h-daily self-administration protocol. The duration of training was enhanced in this experiment in order to allow a longer exposure to the prebiotics that could facilitate their effects to modify the behavioral responses. Mice were trained for six days at FR1, followed by 114 FR5 sessions. As described above, three addiction-like criteria and four phenotypic traits were evaluated at a late period (104-120 sessions). To investigate the role of *Blautia* on the development of food addiction, mice were given lactulose (1%) (n=12) and rhamnose (1%) (n=12) in different groups of mice drinking water during the whole experimental protocol. To avoid possible contamination, water with prebiotics was replaced twice per week, and water intake was monitored. Body weight and food intake were measured once per week.

At the end of the protocol, feces were collected to quantify *Blautia* abundance in the gut by quantitative PCR. DNA was extracted from 0.18-0.2g of feces using QIAamp^{®} Fast DNA Stool Mini kit (Qiagen, Germany) and was stored at -20°C.

To quantify the *Blautia* in the feces, primers g-Blau-R (5'-TTGGTAAGGTTCTTCGCGTT-3') SEQ ID No 3 and g-Blau-F (5'- GTGAAGGAAGAAGTATCTCGG- 3'), targeting SEQ ID No 4 16S rRNA gene, were used, as previously described. Each reaction mixture (10 µl) comprises LightCycler 480 SYBR Green I Master (Roche Life Science, Germany) and 1ng/µl of genomic DNA. The amplification program consisted of one cycle at 95°C for 10 min, followed by 40 cycles at 94°C for 20 s, 55°C for 20 s, and 60°C for 40 s. The fluorescent products were detected in the last step of each cycle. qPCR amplification and detection were performed in 384- well optical plates with an QuantStudio 12K Flex Real-Time PCR System (Applied Biosystems). A melting curve analysis was performed at the end of the experiment to determine the specificity of an assay. The melting program consisted of the first step of 95°C for 15 s, the second step of 60°C for 1 min, and the last dissociation step of 95°C for 15 s. The standard curve for the reference bacterial strain (*Blautia producta,* DSM 2950) was generated using Ct values of 10-fold serial dilutions of genomic DNA. The Ct values in the logarithmic scale were applied to the analytical curve generated in the same experiment to obtain the corresponding bacterial count in each sample. The values were standardized by the weight of feces (grams) at the beginning of DNA extraction.

### Statistical analyses

Statistical analyses of behavioral data were performed with SPSS software (IBM, version 25). Comparisons between the two groups were analyzed by Student t-test or U Mann-Whitney depending on the distribution defined by the Kolmogorov-Smirnov normality test. A p-value <0.05 was used to determine statistical significance. The sample size was calculated based on the power analysis. The criterion for significance (alpha) was set at 0.05, and the statistical test used was a two-sample t-test. With the sample size used, our studies achieved a power superior to 80%. The power analysis showed that sample sizes were similar to those reported in previous publications (Domingo-Rodriguez L, Ruiz de Azua I, Dominguez E, et al. A specific prelimbic-nucleus accumbens pathway controls resilience versus vulnerability to food addiction. Nat Commun 2020;11:1-16. doi:10.1038/s41467-020-14458-).

Principal component analysis (PCA) was performed for dimension reduction prior to the correlational analyses. Correlational analyses were performed between addiction criteria of persistence of response, motivation, and compulsivity in addicted and nonaddicted mice compared to addicted and nonaddicted individuals belonging to the human cohort.

For statistical analyses of gut microbiota in mice, data derived from 16S-sequencing were analyzed using Calypso software (version 8.84) with total sum normalization (TSS) and cumulative-sum scaling (CSS) to account for the non-normal distribution of taxonomic count data for diversity analyses. Alpha diversity was analyzed based on different indexes (Chao1 and Shannon) and their normal distribution. Further differential abundance analyses were performed through DeSeq2, and a linear discriminant analysis effect size (LefSe; with an effect size threshold of 2.0) for searching biomarkers was carried out. Data were considered statistically significant at P<0.05.

In human studies, we excluded those microbial species having less than ten counts in 10% of the samples for metagenomics analyses. Differential abundance analyses for taxa associated with the YFAS scores were performed using the analysis of compositions of microbiomes with bias correction (ANCOMBC) methodology (Lin H, Peddada S Das. Analysis of compositions of microbiomes with bias correction. Nat Commun 2020;11:3514. doi:10.1038/s41467-020-17041-7). It considers the bias due to differential sampling fractions across samples by adding a sample-specific offset to a linear regression model estimated from the observed data. The linear regression model in log scale is analogous to log-ratio transformation to consider the compositional nature of metagenomics datasets, while the offset term serves as the bias correction. All models were adjusted for age, sex, BMI, and years of education. P-values were adjusted for multiple comparisons using the Benjamini Hochberg correction for false discovery rate (pFDR). Statistical significance was set at pFDR<0.1.

### Results

### Characterization of extreme subpopulations of addicted and nonaddicted mice.

genetically homogeneous inbreed C57BI/6J strain of mice that underwent an operant protocol of food addiction during 98 days (Fig. 1a). A total of n=59 mice was trained in operant conditioning maintained by chocolate-flavored pellets (data not shown), and extreme phenotypes of addicted (n=13) and nonaddicted (n=11) mice were selected. In these extreme subpopulations, three hallmarks of addiction were measured during a late training period (from day 78 to 92 of FR5): persistence of response, motivation, and compulsivity. When comparing extreme subpopulations, addicted mice performed higher persistence of response, food motivation, and compulsivity than nonaddicted mice in this late period, as expected (Fig. 1b-d). Furthermore, four phenotypic addiction-related traits were measured, and as expected, the addicted mice showed increased impulsivity, cognitive inflexibility, and appetitive and aversive cue reactivity compared to nonaddicted mice (Fig. 1e-h). In contrast, the number of pellets intake and body weight were similar in addicted and nonaddicted mice. Statistical details are included in Table 1. (n=11 nonaddicted ("NA") mice, n=13 addicted ("A") mice).

**Table 1. Statistical details of the comparison between extreme subpopulations of addicted and nonaddicted mice of Fig. 1**

| Statistical analysis | Factor name | Statistic value | P-value |
|---|---|---|---|
| | Nonaddicted vs addicted | | |
| | Nonaddicted | | |
| | Persistence of response | *K-S=* 0.23 | n.s. |
| | Motivation | *K-S=* 0.32 | p<0.01 |
| | Compulsivity | *K-S=* 0.40 | p<0.001 |
| | Impulsivity | *K-S=* 0.13 | n.s. |
| | Cognitive inflexibility | *K-S=* 0.18 | n.s. |
| | Appetitive cue reactivity | *K-S=* 0.30 | p<0.01 |
| | Aversive cue reactivity | *K-S=* 0.16 | n.s. |
| | Pellet intake | *K-S=* 0.26 | p<0.05 |
| Kol mogorov-Smirnov | Body weight | *K-S= 0.13* | n.s. |
| | Addicted | | |
| | Persistence of response | *K-S=* 0.17 | n.s. |
| | Motivation | *K-S=* 0.22 | n.s. |
| | Compulsivity | *K-S=* 0.30 | p<0.01 |
| | Impulsivity | *K-S=* 0.16 | n.s. |
| | Cognitive inflexibility | *K-S=* 0.16 | n.s. |
| | Appetitive cue reactivity | *K-S=* 0.19 | n.s. |
| | Aversive cue reactivity | *K-S=* 0.22 | n.s. |
| | Pellet intake | *K-S=* 0.17 | n.s. |
| | Body weight | *K-S=* 0.15 | n.s. |
| | Nonaddicted vs addicted | | |
| | Persistence of response | *t*= 4.20 | p<0.001 |
| | Motivation | *U*= 1.50 | p<0.001 |
| | Compulsivity | *U*= 17.50 | p<0.001 |
| U Mann-Whitney or t-test | Impulsivity | *t*= 3.88 | p<0.001 |
| | Cognitive inflexibility | *t*= 3.15 | p<0.01 |
| | Appetitive cue reactivity | *U*= 34 | p<0.05 |
| | Aversive cue reactivity | *t*= 4.04 | p<0.001 |
| | Pellet intake | *U*= 38 | n.s. |
| | Body weight | *t=* 0.49 | n.s. |

### Gut microbiota profile of vulnerability to addiction in mice

We carried out 16S rRNA gene amplicon sequencing of caecum contents to study gut microbiota signatures associated with food addiction-like behavior using the cohorts of extreme phenotypes described in the previous paragraph. As expected, the murine cecal microbiota was dominated by the phyla Firmicutes and Bacteroidetes, reaching almost 90% of the relative abundance, similar in both addicted and nonaddicted mice (Fig. 2a). Alpha-diversity indexes were also similar in both groups (Fig. 2b-c). In contrast, significant differences were observed in bacteria belonging to Actinobacteria (significantly decreased in addicted mice, P<0.01, Fig. 2d), Tenericutes (P<0.01), and Cyanobacteria (P<0.05) phyla, showing the latter increased relative abundances in addicted mice (Fig. 2e,g). Bacteria belonging to the Proteobacteria phylum (p=0.07) had a trend toward increasing levels in the addicted group (Fig. 2f).

Analyses of the data at lower taxonomical levels confirmed these observations. Indeed, the *Coriobacteriaceae* family (from the Actinobacteria phylum) showed lower abundances in the addicted group in comparison to nonaddicted (P<0.05) (Fig. 2i), with lower proportions of *Enterorhabdus* genus (P<0.01) (Fig. 2n) suggesting a potential beneficial effect in the nonaddicted mice. We also observed significant differences in the families and genera's relative abundances of the Bacillota/Firmicutes phylum. Indeed, *Christensenellaceae* (Fig. 2k), *Erysipelotrichaceae* (P<0.05) families (Fig. 2l), and *Lachnospiraceae* UCG-001 genus (P<0.05) (Fig. 2p) had decreased relative abundances in addicted compared with nonaddicted mice, suggesting a protective effect. Other genera such as *Allobaculum* from Bacillota phylum (P<0.05) (Fig. 2q) and *Blautia* genus from Bacillota phylum (P<0.05) (Fig. 2r) showed a similar decrease in addicted mice, further supporting the potential beneficial profile of nonaddicted gut microbiota signatures.

On the other hand, bacteria belonging to the *Anaeroplasmataceae* family (from Tenericutes phylum) (P<0.01) (Fig. 2j), and, consequently, the *Anaeroplasma* genus (Fig 2o), showed higher concentration in addicted mice (P<0.05) in comparison to nonaddicted, suggesting in this case potential non-beneficial roles in the addicted group. Similarly, *Gastranaerophilales* family (from the Cyanobacteria phylum), relative abundances were also increased in addicted mice (P<0.05) (Fig. 2h,m). We also observed significant differences in the families and genera's relative abundances of the Firmicutes phylum. Indeed, *Christensenellaceae* (Fig. 2k), *Erysipelotrichaceae* (P<0.05) families (Fig. 2l), and *Lachnospiraceae* UCG-001 genus (P<0.05) (Fig. 2p) were lower in addicted mice than nonaddicted probably related to a protective gut microbiota profile. Other genera, such as *Allobaculum* from Bacillota phylum (synonym of Firmicutes) (P<0.05) (Fig. 2q) and *Blautia* genus from Bacillota phylum (P<0.05) (Fig. 2r) showed a similar decrease in addicted mice further supporting the potential beneficial profile of nonaddicted gut microbiota signatures. Finally, a microbiome biomarker discovery approach using the linear discriminant analysis effect size method (LEfSe) was applied to compare addicted and nonaddicted groups (Fig. 2s). We observed that *Anaeroplasma, Enterorhabdus,* and *Lachnospiraceae* UCG006 genera were the taxa most likely to explain the differences between addicted and nonaddicted mice groups. Therefore, differential gut microbiota signatures were revealed in addicted and nonaddicted mice despite their similar food intake and similar diet, which suggests a beneficial gut microbiota signature in the nonaddicted groups.

### Gut microbiota correlates with food addiction features in mice

The possible correlations between gut microbiota signatures and the addiction-like criteria and phenotypic traits of the different mouse groups were further investigated (Fig. 3a-b). Interestingly, the non-beneficial *Gastranaerophilales_uncultured organism* group family (from Cyanobacteria phylum), mainly abundant in addicted mice, positively correlated with motivation in this group, whereas it was negatively correlated with motivation in the nonaddicted mice. These results suggest that the potential non-beneficial effect of this bacterium group could be associated with alterations in the motivation for chocolate-flavored pellets. Furthermore, the *Erysipelotrichaceae* family of bacteria (from Firmicutes/Bacillota phylum) correlated negatively with motivation, meaning that addicted mice with high motivation, i.e., the addicted mice with high motivation, showed low *Erysipelotrichaceae* abundance.

The correlation analysis revealed that a cluster of bacteria could affect different addiction criteria. Indeed, the *Gastranaerophilales* bacterium group associated with motivation was also positively correlated with the *Clostridiales_andin BB60* group (from Bacillota/Firmicutes phylum) at the family level in the nonaddicted mice. These bacteria are more abundant in the addicted than in the nonaddicted group, further supporting the non-beneficial effects of this bacteria cluster. The *Clostridiales_vandin 6660 group* was also positively correlated with the persistence of response in the addicted mice. Therefore, both bacterium groups, *Gastranaerophilales* and *Clostridiales_vandin BB60,* are associated with one component of addictive-like behavior, motivation and persistence of response, respectively.

Furthermore, the *Clostridiales_vandin 6660* group positively correlated with the *Ruminococcaceae* family (from Firmicutes phylum), and this family of bacteria was also positively correlated with persistence of response, suggesting a joint action of these clusters of bacteria. Moreover, *Ruminococcaceae* and *Alcaligenaceae* (from the Proteobacteria phylum) were negatively correlated in the addicted group. *Alcaligenaceae* also negatively correlated with inflexibility suggesting positive relationships with better performance. Interestingly, Proteobacteria at the phylum level trends to enhance in addicted mice revealing that not all families of this phylum that integrates the *Alcaligenaceae* show alterations in the same direction.

Those observations were further corroborated at the genus level. Thus, the *Ruminococcaceae_NK4A214_group,* and the *Gastranaerophilales_uncultured organism* genera positively correlated with motivation in the addicted group, whereas the *Clostridiales_vadin 6660 group_uncultured, Erysipelatoclostridium,* and *Parabacteroides genera* negatively correlated with motivation in these addicted mice. Finally, the genera *Ruminococcaceae_UCG009, Lachnospiraceae_FCS020_group, Peptococcus, Candidatus_Arthromitus, Ruminiclostridium_6, Roseburia, Coprococcus_1,* and *Acetatifactor* positively correlated with persistence of response in the addicted group.

### Use of YFAS 2.0 to characterize addicted and nonaddicted humans

The possible gut microbiota signatures associated with food addiction were investigated in a cohort of patients (n=28) classified following the YFAS 2.0 questionnaire. The three addiction-like criteria measured in our food addiction mouse model recapitulates properly the principal features of the food addiction human disease evaluated by the 35-item self-report YFAS 2.0 as reported before (Garcia-Blanco A, Domingo-Rodriguez L, Cabana-Dominguez J, et al. miRNA signatures associated with vulnerability to food addiction in mice and humans. J Clin Invest 2022;132. doi:10.1172/JCI156281). As expected, the sum of the YFAS 2.0 questions, under the criterion of persistence of response, motivation, and compulsivity, was much higher in participants diagnosed with food addiction than in nonaddicted subjects (Fig. 4a-c). In the PCA performed with the main variables (persistence of response, motivation, compulsivity, tolerance, withdrawal, craving, and distress), the two principal components (PC) accounted for 60.5% and 19.2% of variations, respectively (Fig. 4d-g), and two clusters of addicted and nonaddicted groups were separated underlying clear behavioral differences that allow for a distinction of both groups (Fig. 4e). Interestingly, PC1 has strong loadings (>0.7) from all behavioral variables (Fig. 4e-f) except the persistence of response that loads in PC2 (Fig. 4e,g).

Next, we performed a correlation matrix to explore the nature of the association between each addiction variable and phenotypic trait. In agreement with PCA, we observed significant positive correlations between the variables of motivation, compulsivity, tolerance, withdrawal, craving, and distress (>r=0.56) (Fig. 5a), but the persistence of response was less associated with the remaining variables (<r=0.45). A significant correlation was observed between motivation and distress in the correlation matrix (r=0.65, P<0.001), and compulsivity was also strongly associated with distress (r=0.84, P<0.001). These results showed specific relationships between the addiction criteria and the phenotypic traits of distress, craving and withdrawal. It is important to highlight the high positive correlation between compulsivity and motivation (r=0.84, P<0.001), that may represent important biomarkers of pathological behavior.

Finally, we ran the correlation matrix separately for addicted (n=25) and nonaddicted (n=3) individuals and found different correlation patterns in both heatmaps (Fig. 5c,e). In nonaddicted individuals, the majority of correlations were positive or near r=0. However, negative correlations were found in addicted humans between specific variables, such as persistence and motivation (r=-0.99, n.s.), motivation and tolerance (r=-0.99, n.s.), and persistence of response and tolerance (r=1, P<0.05) suggesting unbalanced behavior.

### Cross-characterization of mouse and human behaviors

In mice, we also performed a correlation matrix to explore the nature of the association between each addiction criterion with each phenotypic trait with all the cohort of mice (n=24) and separately with addicted (n=13) and nonaddicted (n=11) mice (Fig. 5b,d,f). In agreement with human results, we observed significant positive correlations between the addiction criteria of motivation and compulsivity (r=0.56, P<0.01), and with the phenotypic traits of cognitive inflexibility and aversive cue-reactivity (>r=0.44, P<0.05) (Fig. 5b), which have a close relationship with craving and distress, respectively. Persistence of response was less associated with the rest of the variables (<r=0.56), but had positive correlation with impulsivity (r=0.63, P<0.001). Significant correlations were also observed between motivation and aversive cue-reactivity (r=0.77, P<0.001), and between compulsivity and appetitive cue-reactivity (r=0.64, P<0.001) similarly to the correlation found in humans between compulsivity and craving (r=0.75, P<0.001). These results showed the crucial translational value of the data. Furthermore, the majority of correlations were positive in the nonaddicted mice (Fig. 5d), whereas there was a predominance of negative correlations in the addicted mice (Fig. 5f), parallel to the results obtained in humans.

Correlational analyses between addiction criteria in mice and humans were also analyzed together (Fig. 5g-i). A strong negative correlation between compulsivity and persistence of response was found for addicted individuals and a moderate positive correlation for nonaddicted (both humans and mice). The slope is highly similar for mice and humans and follows the same correlation pattern (Fig. 5g). In the correlation between compulsivity and motivation, a mild negative correlation for addicted individuals (mice) was found, while positive correlations for nonaddicted individuals (mice and humans) and addicted humans were described (Fig. 5h). In the correlation between motivation and persistence of response, a negative correlation for addicted individuals (humans and mice) was found together with a moderate positive correlation for nonaddicted individuals (mice and humans) with a similar slope (Fig. 5i). These similar correlations found in mice and humans further underline the translational value of our behavioral results.

### Gut microbiota signatures in humans

Differential bacterial abundance using ANCOM-BC controlling for age, BMI, and sex in humans (n=28) were observed in the volcano plot. Microbiota signatures, distinct between addicted and nonaddicted individuals, are represented by the differential expression of bacteria species taxa colored according to the phylum (Fig. 6). Multiple similitudes were found to be overlapped between mice and humans. Interestingly, *Blautia producta* species was increased in individuals with a low score in the YFAS scale (Fig. 6), in agreement with the decreased *Blautia* genus abundance in addicted mice (Fig. 2r). Furthermore, we observed significant differences in humans in the relative abundances of bacteria belonging to Actinobacteria, Bacillota/Firmicutes, and Proteobacteria phyla that were in the same line as what was found in the mice cohort. From the Actinobacteria phylum, the abundance of the *Actinomyces bouchesdurhonensis, Bifidobacterium dentiu,* and *Adlercreuzia muris* species were increased in nonaddicted individuals, in parallel to what was found in mice at the genus level. Furthermore, the species *Erysipelatoclostridium ramosum* (from *Erysipelotrichaceae* family and Bacillota/Firmicutes phylum) was also increased in humans with a low score in the YFAS, similar to what was described for mice at the family level and suggesting a potentially beneficial effect. Concerning Proteobacteria phylum, higher concentration was found in individuals diagnosed with addiction, specifically in several species, such as *Burkholderiaceae unclassified, Zoogloeaceae unclassified, Rhizobium etli, Zoogloea, Lawsonia intracellularis, and Parasutterella,* among others with coherence to the results in mice that suggested a non-beneficial effect for this phylum.

### Functional validation with prebiotics in the mouse food addiction protocol

Both mice and human results suggest that some specific microbiota could be protective in preventing food addiction. The strong similitudes found in the *Blautia* genus content in both species underline the potential beneficial effects of this particular gut microbiota. However, *Blautia* is a strictly anaerobic bacteria, and its possible therapeutical use as a probiotic to prevent food addiction would be difficult. Interestingly, several well-known prebiotics that could have possible straight use in humans have been reported to enhance *Blautia* genus content. Therefore, we have investigated the possible protective effects promoted by oral administration of lactulose and rhamnose as prebiotics able to enhance Blautia genus abundance in the gut.

For this purpose, a total of 36 C57BI/6J mice underwent an operant protocol of 120 sessions (Fig. 7a). The duration of training was increased for more prolonged exposure to the prebiotics that could facilitate their effects to modify the behavioral responses. All groups significantly increased the number of reinforcers across the FR1 and FR5 sessions, with slight variation between groups, indicating similar levels of primary reinforcement of chocolate-flavored pellets. In the late period (95-112 sessions). A mouse was considered positive for an addiction-like criterion when its score for each behavior was equal to or further than the 75th percentile of the distribution of the control group as was previously reported (Garcia-Blanco A, Domingo-Rodriguez L, Cabana-Dominguez J, et al. miRNA signatures associated with vulnerability to food addiction in mice and humans. J Clin Invest 2022;132. doi:10.1172/JCI156281). Notably, 41.67% of control mice achieved 2-3 criteria and were considered addicted, whereas none of the mice receiving lactulose or rhamnose achieved the addiction criteria (χ²=8.57, control vs lactulose and χ²=12, control vs rhamnose P<0.01, Fig. 7e). Mice treated with lactulose or rhamnose showed similar persistence of response to the control group (Fig. 7b). In contrast, motivation for chocolate-flavored pellets was reduced in the rhamnose group compared to lactulose (Mann-Whitney's U=31.50, P<0.05, Fig. 7c). Importantly, mice receiving rhamnose showed decreased compulsivity in seeking for palatable food compared to control mice, revealing a beneficial effect of this prebiotic to prevent food addiction (Mann-Whitney's U=31.50, P<0.01, Fig. 7d). Four additional phenotypic traits considered as factors of vulnerability to addiction, impulsivity, cognitive inflexibility, appetitive cue reactivity and aversive cue reactivity, were also evaluated (Fig. 7f-i). No significant differences among groups were found in impulsivity, appetitive, or aversive cue reactivity. Interestingly, cognitive inflexibility was lower in rhamnose mice compared to control (Mann-Whitney's U=35.50, P<0.05) and lactulose (Mann-Whitney's U=17.00, P<0.001) groups in coherence with the protective effect of this prebiotic (Fig. 7g). All mice had similar food and water intake as well as similar body weight during the whole experimental sequence (Fig. 7j-l).

A qPCR was performed to confirm the effectiveness of targeted prebiotics in increasing *Blautia* genus abundance in the gut. Based on the generated standard curve, the amount of *Blautia* per gram of feces was determined. No PCR inhibition or nonspecific binding was observed in the assay. Our results revealed that mice receiving rhamnose had significantly increased *Blautia* abundance in the gut (P<0.01, Fig. 7m), whereas only a non-significant trend to enhance this genus was shown in mice receiving lactulose. Statistical details are included in Table 2.

**Table 2: Statistical details.**

| **Statistical analysis** | **Factor name** | **Statistic value** | **P-value** |
|---|---|---|---|
| Repeated measures ANOVA | FR1 sessions | *F(5,165)=9,59* | p<0.001 |
| | FR1 sessions x treatment | *F(10, 165)=2.84* | p<0.01 |
| | FR5 sessions | *R(98, 1960)=7.93* | p<0.001 |
| | FR5 sessions x treatment | *F(196*, *1960*)=*1.44* | p<0.001 |
| | **Control vs lactulose vs rhamnose** | | |
| | **Control** | | |
| | Persistence of response | K-S=0.15 | n.s. |
| | Motivation | K-S=0.20 | n.s. |
| | Compulsivity | K-S=0.22 | n.s. |
| | Impulsivity | K-S=0.29 | p<0.01 |
| | Cognitive inflexibility | K-S=0.25 | p<0.05 |
| | Appetitive cue reactivity | K-S=0.24 | p<0.05 |
| | Aversive cue reactivity | K-S=0.25 | p<0.05 |
| | Pellet intake | K-S=0.25 | p<0.05 |
| | Body weight | K-S=0.24 | n.s. |
| | Water intake | K-S=0.12 | n.s. |
| | **Lactulose** | | |
| | Persistence of response | K-S=0.23 | n.s. |
| | Motivation | K-S=0.25 | p<0.05 |
| | Compulsivity | K-S=0.20 | n.s. |
| | Impulsivity | K-S=0.19 | n.s. |
| Kolmogorov-Smirnov | Cognitive inflexibility | K-S=0.29 | p<0.01 |
| | Appetitive cue reactivity | K-S=0.24 | n.s. |
| | Aversive cue reactivity | K-S=0.13 | n.s. |
| | Pellet intake | K-S=0.13 | n.s. |
| | Body weight | K-S=0.17 | n.s. |
| | Water intake | K-S=0.12 | n.s. |
| | **Rhamnose** | | |
| | Persistence of response | K-S=0.12 | n.s. |
| | Motivation | K-S=0.21 | n.s. |
| | Compulsivity | K-S=0.43 | p<0.001 |
| | Impulsivity | K-S=0.28 | p<0.01 |
| | Cognitive inflexibility | K-S=0.178 | n.s. |
| | Appetitive cue reactivity | K-S=0.16 | n.s. |
| | Aversive cue reactivity | K.S=0.20 | n.s. |
| | Pellet intake | K-S=0.11 | n.s. |
| | Body weight | K-S=0.10 | n.s. |
| | Water intake | K-S=0.19 | n.s. |

**Table 2: Continuation.**

| | | | |
|---|---|---|---|
| | **Control vs lactulose** | | |
| | Persistence of response | t=0.01 | n.s. |
| | Motivation | U=65 | n.s. |
| | Compulsivity | t=1.45 | n.s. |
| | Impulsivity | U=64.5 | n.s. |
| | Cognitive inflexibility | U=71.5 | n.s. |
| | Appetitive cue reactivity | U=64.5 | n.s. |
| U Mann-Whitney or t-test | Aversive cue reactivity | U=64.5 | n.s. |
| | Pellet intake | U=40 | n.s. |
| | Body weight | t=1.1 | n.s. |
| | Water intake | t=2.13 | p<0.05 |
| | **Control vs rhamnose** | | |
| | Persistence of response | t=0.92 | n.s. |
| | Motivation | t=1.74 | n.s. |
| | Compulsivity | U=31.50 | p<0.05 |
| | Impulsivity | U=44 | n.s. |
| | Cognitive inflexibility | U=35.5 | p<0.05 |
| | Appetitive cue reactivity | U=53.5 | n.s. |
| | Aversive cue reactivity | U=62 | n.s. |
| | Pellet intake | U=68 | n.s. |
| | Body weight | t=1.07 | n.s. |
| | Water intake | t=1.38 | n.s. |
| | **Lactulose vs rhamnose** | | |
| | Persistence of response | t=0.97 | n.s. |
| | Motivation | U=32.5 | p<0.05 |
| | Compulsivity | U=41.50 | n.s. |
| | Impulsivity | U=37 | p<0.05 |
| | Cognitive inflexibility | U=17 | p<0.001 |
| | Appetitive cue reactivity | t=0.77 | n.s. |
| | Aversive cue reactivity | t=1.15 | n.s. |
| | Pellet intake | t=-0.766 | n.s. |
| | Body weight | t=-0.384 | n.s. |
| | Water intake | t=-0.704 | n.s. |
| Chi-Square | Control vs lactulose | X2= | p<0.001 |
| | Control vs rhamnose | X2= | p<0.001 |
| Kolmogorov-Smirnov | **Control vs lactulose vs rhamnose** | | |
| | **Control** | | |
| | Concentration/g feces | *K-S=* 0.26 | p<0.05 |
| | **Lactulose** | | |
| | Concentration/g feces | *K-S=* 0.23 | n.s. |
| | **Rhamnose** | | |
| | Concentration/g feces | *K-S=* 0.27 | p<0.05 |
| U Mann-Whitney | **Control vs lactulose** | | |
| | Concentration/g feces | *U=59* | n.s. |
| | **Control vs rhamnose** | | |
| | Concentration/g feces | *U=30* | p<0.05 |
| | **Lactulose vs rhamnose** | | |
| | Concentration/g feces | *U=46* | n.s. |

A PCA was performed to further understand the correlation between prebiotics administration and behavioral improvement leading to the prevention of food addiction. The percentage of variance explained by the two PC was 33.2% (PC1) and 22.5% (PC2). Mice classified as addicted and nonaddicted clustered separately between groups and together within groups (Fig. 8a). Interestingly, those mice receiving rhamnose clustered together with the nonaddicted control mice confirming the protective effects of this prebiotic. Moreover, lactulose mice also cluster close to the nonaddicted control mice suggesting a possible potential beneficial effect of this second prebiotic. The main variables loading in PC1 were cognitive inflexibility, the persistence of response, and impulsivity (>0.7), while aversive cue reactivity and compulsivity were the main criteria loading in PC2 (>0.7) (Fig. 8b-d). Impulsivity participates more in the first component, whereas compulsivity is more critical in the second component (Fig. 8c-d), resembling the sequential feature of the transition from impulsivity to compulsivity described in addiction. Compulsivity and persistence of response are loaded separately in the two PCs, similarly to the human results.

When representing the addiction-like criteria and phenotypic traits into a heatmap of correlations among all mice (n=36), we observed significant positive correlations between the criteria of motivation with the impulsivity and cognitive inflexibility traits (r=0.43, P<0.01) (Fig. 8e). The persistence of response criterion was less associated with the rest of the variables (<r=0.12), but had a positive correlation with impulsivity (r=0.39, P<0.05) and cognitive inflexibility (r=0.58, P<0.001). Compulsivity was the addiction criterion primarily associated with the addiction criteria score (r=0.56, P<0.001). Interestingly, appetitive cue reactivity showed negative correlations with all variables related to prebiotic protective effects (<r=-0.11, n.s.). *Blautia* genus had a negative correlation with addiction criteria score (r=-0.32, n.s.), further emphasizing its beneficial effects.

### Combined preparation of rhamnose and lactulose

An identical experiment was performed but mice were fed with a solution of 1% w/w of each prebiotic, or a combination of both of them under the same experimental conditions following the same steps of *Functional validation with prebiotics in the mouse food addiction protocol.* (Fig. 9) The alleviation of the symtomps of food addiction were much better than when lactulose and rhamnose administered separately, therefore demonstrating the synergistic effect of the combination

### List of sequences

SEQ ID No 1: F (5'-

TCGTCGGCAGCGTCAGATGTGTATAAGAGACAGCCTACGGGNGGCWGCAG-3')

SEQ ID No 2: (5’-
GTCTCGTGGGCTCGGAGATGTGTATAAGAGACAGGACTACHVGGGTATCTAATCC-
3'))]

SEQ ID No 3: g-Blau-R (5'-TTGGTAAGGTTCTTCGCGTT- 3')
SEQ ID No 4: g-Blau-F (5'- GTGAAGGAAGAAGTATCTCGG- 3')

## Claims

1. A combined preparation of rhamnose and lactulose for use in the treatment and/or prevention of food addiction in a subject.

2. The combined preparation for use, according to the preceding claim, wherein the administration to the subject is simultaneous, sequential or separate, preferably simultaneous.

3. The combined preparation for use, according to any of the preceding claims, wherein the amount of lactulose and rhamnose is approximately the same.

4. The combined preparation for use, according to any of the preceding claims, dissolved in water.

5. The combined preparation for use, according to the preceding claim, wherein lactulose is from 0.1 to 10% w/w in water and rhamnose is from 0.1 to 10% in water

6. The combined preparation for use, according to any of the preceding claims, by oral administration once per day for a period of time between 30 days and 120 days.

7. A method to detect food addiction in a subject comprising the detection of an amount of bacterial strains selected from *Enterorhabdus, Lachnospiracceae, Allobaculum,* and *Blautia* from a first biological sample, wherein the amount of the bacterial strains of *Enterorhabdus, Lachnospiracceae, Allobaculum,* and *Blautia* is reduced as compared to a reference value

8. The method according to the preceding claim wherein bacterial strains of *Blautia* are one or more of the following *Blautia producta, Blautia glucerasea, Blautia schinkii* and *Blautia wexlerae*

9. The method according to any of the preceding claims 7 or 8, wherein the reference value is the bacterial amount of *Enterorhabdus, Lachnospiracceae, Allobaculum* in a biological sample of a subject with a body mass index between 18.5 and 29.9 kg/m², or is the bacterial amount obtained in a second biological sample from the same subject, said biological sample is obtained before the first biological sample

10. The method according to any of the preceding claims 7 to 9, comprising measuring the amount of bacterial strains selected from the genera of *Anaeroplasma* and *Gastranaerophilales* from the first biological sample, wherein the amount of the bacterial strains of said genera is increased as compared to a reference value

11. The method according to the preceding claim, wherein the reference value is the bacterial amount of *Anaeroplasma* and *Gastranaerophilales* in a biological sample of a subject with a body mass index between 18.5 and 29.9 kg/m², or is the bacterial amount obtained in a second biological sample from the same subject, said biological sample is obtained before the first biological sample

12. The method according to any of the preceding claims 7 to 11, wherein the biological sample is a sample of feaces.

13. The combined preparation, for use according to any of the preceding claims 1 to 6, wherein food addiction is detected according to the method of any of the claims 7 to 9.

14. The combined preparation, for use according to the preceding claims 1 to 6, wherein food addiction is detected according to the method of any of the claims 7 to 12.

15. Kit to perform the method described in any of the claims 7 to 12 comprising the primer pair formed by SEQ ID No 1 and SEQ ID no 2 and/or a variant having at least 80%, preferably at least 85%, more preferably at least 90 % and even more preferably 91 % or 92 % or 93 % or 94 % or 95 % or 96 % or 97 % or 98 % or even up to 99 % identity with respect to SEQ ID No 1, and/or SEQ ID No 2.
